# EUROPEAN PATENT SPECIFICATION

(11) **EP 1 432 446 B1**
(45) Date of publication and mention of the grant of the patent: **27.04.2011**
(21) Application number: 02797854.3
(22) Date of filing: 04.09.2002
(51) Int. Cl.: A61K 39/395, C07K 16/00, C12P 21/08, C12Q 1/68, G01N 33/53, G01N 33/574, A61P 35/00

(54) **METHODS FOR THE IDENTIFICATION OF POLYPEPTIDE ANTIGENS ASSOCIATED WITH DISORDERS INVOLVING ABERRANT CELL PROLIFERATION**
VERFAHREN ZUR IDENTIFIKATION VON POLYPEPTID-ANTIGENEN IM ZUSAMMENHANG MIT STÖRUNGEN DURCH ABERRANTE ZELLPROLIFERATION
METHODES D'IDENTIFICATION D'ANTIGENES DE POLYPEPTIDES LIES A DES TROUBLES SE TRADUISANT PAR UNE PROLIFERATION CELLULAIRE ABERRANTE

(30) Priority: 05.09.2001 US 317504 P
(43) Date of publication of application: 30.06.2004
(73) Proprietor: Genentech, Inc., South San Francisco CA 94080-4990 (US)
(72) Inventor: LEVINSON, Arthur D., South San Francisco, CA 94080 (US)
(74) Representative: Walton, Seán Malcolm
(86) International application number: PCT/US2002/028176
(87) International publication number: WO 2003/020909

(56) References cited:
- EP-A- 0 425 235
- WO-A-00/52054
- WO-A-01/40309
- MOCH H ET AL: "HIGH-TROUGHPUT TISSUE MICROARRAY ANALYSIS TO EVALUATE GENES UNCOVERED BY CDNA MICROARRAY SCREENING IN RENAL CELL CARCINOMA" AMERICAN JOURNAL OF PATHOLOGY, PHILADELPHIA, PA, US, vol. 154, no. 4, January 1999 (1999-01), pages 981-986, XP002934470 ISSN: 0002-9440
- COOPER C S: "Applications of microarray technology in breast cancer research" BREAST CANCER RESEARCH, CURRENT SCIENCE, LONDON, GB, vol. 3, no. 3, 20 March 2001 (2001-03-20), pages 158-175, XP002963070 ISSN: 1465-5411
- SCHMIDT M ET AL: "SYNERGISTIC INTERACTION BETWEEN AND ANTI-P185HER-2 PSEUDOMONAS EXOTOXIN FUSION PROTEIN SCFV(FRP5)-ETA AND LONIZING RADIATION FOR INHIBITING GROWTH OF OVARIAN CANCER CELLS THAT OVEREXPRESS HER-2" GYNECOLOGIC ONCOLOGY, ACADEMIC PRESS, LONDON, GB, vol. 80, no. 2, February 2001 (2001-02), pages 145-155, XP001024046 ISSN: 0090-8258
- LIU C ET AL: "ERADICATION OF LARGE COLON TUMOR XENOGRAFTS BY TARGETED DELIVERY OFMAYTANSINOIDS" August 1996 (1996-08), PROCEEDINGS OF THE NATIONAL ACADEMY OF SCIENCES OF USA, NATIONAL ACADEMY OF SCIENCE. WASHINGTON, US, PAGE(S) 8618-8623 , XP002938232 ISSN: 0027-8424 * the whole document *
- CHARI R V J ET AL: "IMMUNOCONJUGATES CONTAINING NOVEL MAYTANSINOIDS PROMISING ANTICANCER DRUGS" CANCER RESEARCH, vol. 52, no. 1, 1992, pages 127-131, XP000453560 ISSN: 0008-5472
- SMITH S: "Technology evaluation: C242-DM1, ImmunoGen Inc." CURRENT OPINION IN MOLECULAR THERAPEUTICS. APR 2001, vol. 3, no. 2, April 2001 (2001-04), pages 198-203, XP008011026 ISSN: 1464-8431
- LEWIS ET AL.: 'Differential responses of human tumor cell lines to anti-p 185HER2 monoclonal antibodies' CANCER IMMUNOL. IMMUNOTHER. vol. 37, no. 4, September 1993, pages 255 - 263, XP002961547
- BULL ET AL.: 'Identification of potential diagnostic markers of prostate cancer and prostatic intraepithelial neoplasia using cDNA microarry' BRITISH JOURNAL OF CANCER vol. 84, no. 1, 01 June 2001, pages 1512 - 1519, XP001015792
- LIU ET AL.: 'Eradication of large colon tumor xenografts by targeted delivery of maytansinoids' PROC. NATL. ACAD. SCI. USA vol. 93, August 1993, pages 8618 - 8623, XP002938232
- LIU ET AL.: 'The development of antibody delivery systems to target cancer with highly potent maytansinoids' EXP. OPIN. INVEST. DRUGS vol. 6, no. 2, 1997, pages 169 - 172, XP001030722
- CHARI ET AL.: 'Dose-response of the anti-tumor effect of huN901-DM1 against human small-cell lung cancer xenografts' PROC. AM. ASSOC. CANCER RES. vol. 41, 2000, page 693, ABSTRACT 4405, XP002961546
- SLIWKOWSKI ET AL.: 'Nonclinical studies addressing the mechanism of action of trastuzumab (Herceptin)' SEMIN ONCOL. vol. 26, no. 4, SUPPL. 12, August 1999, pages 60 - 70, XP002961545
- RANSON ET AL.: 'Perspectives on anti-HER monoclonal antibodies' ONCOLOGY vol. 63, no. SUPPL. 1, 2002, pages 17 - 24, XP002961544
- SMITH: 'Technology evaluation: C242-DM1, immunogen inc.' CURR. OPIN. MOL. THER. vol. 3, no. 2, April 2001, pages 198 - 203, XP008011026

## Description

### 1. Field of the Invention

The present invention relates to methods that are useful for the identification of polypeptide antigens that are associated with disorders involving aberrant cell proliferation (*e.g*., cancer). More specifically, the invention relates to novel methods for the identification of cellular polypeptide antigens which serve as effective targets for cancer therapy.

### 2. Background of the Invention

### 2.1. Cell Mitosis

Cell mitosis is a multi-step process that includes cell division and replication (Alberts, B., et al., In The Cell, pp. 632-661 (1989); Sayer, E. Biochemistry (1988)). Cells reproduce by division into two daughter cells. The DNA replication phase of the cell reproduction cycle is known as the "S phase", During the S-phase, chromosomes within a cell are replicated, yielding pairs of identical daughter DNA molecules known as sister chromatids, which then separate during mitosis to produce two new nuclei. Although the term "mitosis" is commonly used synonomously with the term "cell division", mitosis correctly refers to only one phase of the cell division process: the process in which the sister chromatids are partitioned equally between the two daughter cells. In eukaryotic cells, mitosis is followed by cytokinesis, which is the process by which the cell cytoplasm is cleaved into two distinct but genetically identical daughter cells.

At the onset of mitosis, small intracellular filamentous structures known as cytoplasmic microtubules, of which the major component is a protein called tubulin, disassemble into tubulin molecules. The tubulin then reassembles into microtubules forming an intracellular structure known as the "mitotic spindle". The mitotic spindle plays a critical role in distributing chromosomes within the dividing cell precisely between the two daughter nuclei. As such, it is clear that the formation of intracellular microtubules is an essential step in the mammalian cell proliferation process.

Unfortunately, however, numerous diseases are characterized by abnormal cell proliferation. As one example, uncontrolled cell division is a hallmark of cancer. Cancer is the leading cause of death, second only to heart disease, of both men and women. In the fight against cancer, numerous techniques have been developed aud are the subject of current research directed to understanding the nature and cause of the disease and to providing methods for the control or cure thereof.

Cancer cells are generally characterized by more rapid cell division and proliferation than observed in most healthy cells, and many anti-cancer agents operate by inhibiting cell division. Since cancer cells divide more rapidly than do healthy cells, cancer cells are preferentially killed by anti-cancer agents which inhibit mitosis. Such compounds often are called "antimitotic" compounds.

### 2.2. Anti-Tumor Agents

To date, three major families of antitumor agents are known. Each of the families of agents is associated with a recognized mechanism of action. First, antitumor agents may be alkylating agents, which generally bind in a covalent manner with DNA to form bifunctional lesions. The bifunctional lesions involve adjacent or nearby bases of the same strand or, alternatively, involve bases on opposite strands forming interstrand crosslinks. Examples of alkylating agents include nitrogen mustard, cyclophosphamide and chlorambucil. Toxicities associated with the use of alkylating agents include nausea, vomiting, alopecia, hemorrhagic cystitis, pulmonary fibrosis, etc. Second, antitumor agents may be antimetabolites, which generally inhibit enzymes involved in the synthesis or assembly of DNA. Alternatively, an antimetabolite may serve as a fraudulent or analog substrate of DNA processes. Examples of antimetabolites include purine, pyrimidine and folate antagonists and plant alkaloids such as vincristine and vinblastine. Toxicities associated with the use of antimetabolites include alopecia, myelosuppression, vomiting, nausea, peripheral neuropathy, etc. Third, antitumor agents may be antibiotics, which work by intercalating into the DNA helix or introducing strand breaks into DNA. Examples of antibiotics include doxorubicin, daunorubicin and actinomycin. Toxicities associated with the use of antibiotics include myelosuppression, anaphylactic reactions, anorexia, cardiotoxicity, pulmonary fibrosis, etc.

Several classes of antimitotic compounds are known which, when administered to dividing cells, prevent the formation of the mitotic spindle by binding to tubulin or microtubules. Absence of a mitotic spindle results in the arrest of mitosis and an accumulation of cells with visible sister chromatids, but without normal mitotic figures. Inability of the cells to divide ultimately results in cell death. Such compounds are discussed in, for example, E. Hamel, Medicinal Research Reviews, vol. 16, pp. 207-231 (1996). Examples of compounds which are known to prevent the formation of a mitotic spindle include the Catharalthus alkaloids vincristine and vinblastine; benzimidazole carbamates such as nocodazole; colchicine and related compounds such as podophyllotoxin, steganacin and combretastatin; taxanes such as paclitaxel and docetaxel; and maytansinoids. The alkaloids, vincristine and vinblastine, the taxane-based compounds and maytansinoids have been used as anticancer drugs (see, for example, E. K. Rowinsky and R. C. Donehower, Pharmacology and Therapeutics, vol. 52, pp. 35-84 (1991)).

Ionizing radiation also is a well established treatment for malignant disease and is ofproven benefit for both curative and palliative purposes. However, radiotherapy can have several undesirable complications, such as mucositis, leukopenia, desquamation, spinal cord necrosis and obliterative endarteritis. These complications frequently limit the ability to deliver a full therapeutic dose of radiation or cause significant morbidity following treatment. Many chemotherapy agents are also toxic to cells of normal tissue, and, thus, the side-effects of chemotherapy are sometimes almost as devastating to the patient as the tumor burden itself. One approach to reducing the side effects of chemotherapy has been to attempt to target chemotherapeutic agents, including radioisotopes and various plant and bacterial toxins, to tumor cells by attaching the agents to antibodies that are specific for antigens present on a tumor cell. See, e.g., U.S. Pat. Nos. 4,348,376 and 4,460,559 which describe radioimmunotherapy of solid tumors (carcinomas) using an anti-carcinoembryonic antigen antibody, and U.S. Pat. No. 5,595,721 which is directed to radioimmunotherapy of lymphoma, a more disseminated tumor. However, the results of therapy using antibody conjugates generally has been disappointing. Remission rates have been low and generally non-reproducible.

### 2.3. Maytansinoids

Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and maytansinol analogues are well known in the art and disclosed, for example, in U.S. Patent Nos. 4,137,230; 4,248.870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

Maytansine and maytansinoids are highly cytotoxic but their clinical use in cancer therapy has been greatly limited by their severe systemic side-effects, primarily attributed to their poor selectivity for tumors. Clinical trials with maytansine had been discontinued due to serious adverse effects on the central nervous system and gastrointestinal system (Issel et al., 1978, Can. Trtmnt. Rev., 5:199-207). Moreover, maytansine was found to be associated with peripheral neuropathy. As such, the use of maytansinoids in cancer therapy has provided only limited success.

### 2.4. Maytansinoids Conjugated With Antibodies

In an attempt to improve their therapeutic index, maytansine and maytansinoids have been conjugated to antibodies specifically binding to tumor cell antigens. More specifically, therapeutic modalities have involved conjugating maytansinoids to antibodies which recognize and bind to antigens present on tumor cells but not on normal cells, thereby limiting the toxic effects of the maytansinoids to only the tumor cells. Examples of such immunoconjugates containing maytansinoids which bind tumor cell antigens are disclosed, for example, in U.S. Patent Nos. 5,208,020; 5,416,064 and European Patent EP 0 425 235 B1, the disclosures of Liu et al., Proc. Natl. Acad Sci. USA. 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an *in vivo* tumor growth assay. In fact, the C242-DM1 conjugate was cytotoxic not only to about 100% of COLO 205 cells, of which the C242 antigen (CanAg) is expressed on about 100% of the cells, but also to about 99% of LoVo cells, of which only about 20-30% express the C242 antigen. Chari et al., Cancer Research, 52:127-131 (1992) describe immunoconjugates in which a maytansinoid was conjugated via a disulfide linker to the murine antibody A7 binding to an antigen on human colon cancer cell lines, or to another murine monoclonal antibody TA. 1 that binds the HER-2/neu oncogene. The cytotoxicity of the TA.1-maytansonoid conjugate was tested *in vitro* on the human breast cancer cell line SK-BR-3, which expresses 3x10⁵ HER-2 surface antigens per cell. The drug conjugate achieved a degree of cytotoxicity similar to the free maytansonid drug, which could be increased by increasing the number of maytansinoid molecules per antibody molecule. The A7-maytansinoid conjugate showed low systemic cytotoxicity in mice. Many of the above-described maytansinoid-antibody conjugates utilize 3-4 maytansinoid molecules per antibody molecule, thereby increasing the cytotoxicity of the conjugate.

Although the above described maytansinoid-antibody conjugates have provided some success, overall therapeutic efficacy has been limited because it has proven to be extremely difficult to identify and produce antibodies which bind specifically only to tumor cell antigens, and not to antigens on all normal cells. As such, previously employed methods and compositions using maytansinoids have resulted in relatively high levels of general toxicity to normal cells in the body. Therefore, there exists a need for novel methods which would be useful for identifying cellular polypeptide targets for cell mitosis inhibitors that are not subject to the general toxicity described above.

### 2.5. Need for Further Treatments

Although thousands of potential anticancer agents have been evaluated, the treatment of human cancer remains fraught with complications which often present an array of suboptimnal treatment choices. As such, chemotherapeutic agents which possess little or no toxicity, which are inexpensive to obtain or manufacture, which are well tolerated by the patient, and which are easily administered would be a desirable addition to the therapeutic modalities currently available to the oncologist. Agents that will selectively sensitize malignant tissue to allow lower doses of radiation or therapy to achieve the same therapeutic effect with less damage to healthy tissues are also desirable. Similarly, agents that prevent cancer from occurring or reoccurring are also desirable. The present invention overcomes deficiencies of current antibody-toxin conjugate modalities in that it is not essential to utilize antibodies which specifically bind to antigens on the surface of tumor cells but not on all normal cells.

### 3. Summary of the Invention

The scope of the present invention is defined by the claims and any information that does not fall within the claims is provided for information described herein are only methods for the development of effective cancer therapies using mitotic inhibitors which have limited general toxicity to normal, non-cancerous, cells and tissues of a patient. The methods utilize cytotoxic compounds comprised of an antibody conjugated to an anti-mitotic compound. The antibodies are substantially incapable of inducing antibody-dependent cell-mediated cytotoxicity (ADCC) and/or complement dependent cytotoxicity (CDC), thereby ensuring that the therapeutic effect (*e.g*., cell killing of only proliferating cells) is mediated primarily by the anti-mitotic component of the cytotoxic compound, rather than by indirect cell killing via ADCC and/or CDC.

Described herein are methods and compositions for the treatment of disorders involving aberrant cell proliferation (*e.g*., cancer). More specifically, the invention is based on the discovery that compounds which exhibit anti-mitotic properties (*e.g*., maytansinoids), when conjugated to a cell binding agent (*e.g*., antibodies), are effective at treating disorders characterized by increased cell proliferation. The present invention is based on the surprising discovery that the cell binding agent does not need to be specific for tumor cell antigens (*i.e*., present on tumor cells but not on normal cells). Rather, the cell binding agent need only differentiate between polypeptide antigens which are more highly expressed on proliferating cancer cells as compared to proliferating non-cancer cells,

The invention provides a method for identifying a polypeptide antigon on the surface of a cell which may be used as a target for cancer therapy. In an additional embodiment, the step of identifying a polypeptide antigen comprises employing microarray analysis.

### 4. Brief Description of the Drawings

Figure 1 shows the structure of a maytansinoid, designated "DM 1." In the structure of DM 1, "R" can be occupied by a variety of groups capable of forming a chemical bond with a selected linker. Preferably, "R" is an SH group or a protected derivative thereof, which forms an S-S bond with a linker, such as N-succinimidyl-4-(2-pyridylthio)pentanoate (SPP).
Figure 2 illustrates the structure of a HERCEPTIN^{®}-DM1 conjugate.
Figure 3 is the elution profile of HERCEPTIN^{®}-DM1 conjugate on a Sephacryl S300 gel filtration column.
Figure 4 shows the anti-proliferative effect of HERCEPTIN^{®} and HERCEPTIN^{®}-DM1 conjugate on SK-BR3 cells *in vitro.* As control, the unrelated monoclonal antibody RITUXAN^{®} or RITUXAN^{®}-DM1 conjugate was used.
Figures 5 (A-D) show that normal human cells (human mammary epithelial cells (5A); human hepatocytes (5B); normal human epidermal keratinocytes (5C); and small airway epithelial cells (5D)) are not killed by HERCEPTIN^{®}-DM1 conjugates.
Figures 6 (A-B) show that growth-arrested cells are insensitive to HERCEPTIN^{®}-DM1,

### 5. Detailed Description of the Invention

The scope of the present invention is defined by the claims and any information that does not fall within the claims is provided for information only.

### 5.1. Definitions

The terms "cancer", "cancerous", and "malignant" refer to or describe the physiological condition in mammals that is typically characterized by unregulated cell growth. Examples of cancer include but are not limited to, carcinoma including adenocarcinoma, lymphoma, blastoma, melanoma, sarcoma, and leukemia. More particular examples of such cancers include squamous cell cancer, small-cell lung cancer, non-small cell lung cancer, gastrointestinal cancer, Hodgkin's and non-Hodgkin's lymphoma, pancreatic cancer, glioblastoma, cervical cancer, ovarian cancer, liver cancer such as hepatic carcinoma and hepatoma, bladder cancer, breast cancer, colon cancer, colorectal cancer, endometrial carcinoma, salivary gland carcinoma, kidney cancer such as renal cell carcinoma and Wilms' tumors, basal cell carcinoma, melanoma, prostate cancer, vulval cancer, thyroid cancer, testicular cancer, esophageal cancer, and various types of head and neck cancer. The preferred cancers for treatment herein are breast, colon, lung, melanoma, ovarian, and others involving vascular tumors as noted above.

The term "cytotoxic agent" as used herein refers to a substance that inhibits or prevents the function of cells and/or causes destruction of cells. The term is intended to include radioactive isotopes (*e.g*. At²¹¹, 1¹³¹, 1¹²⁵, Y^{90,} Re¹⁸⁶, Re¹⁸⁸, Sm¹⁵³, Bi²¹², P³² and radioactive isotopes ofLu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such, as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and cyclosphosphamide (CYTOXAN^{™}); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as cannustine, chlorozotocin, fotemustine, lomustine, nimustine, ranimustine; antibiotics such as aclacinomysins, actinomycin, authramycin, azaserine, bleomycins, cactinomycin, calicheamicin, carabicin, carminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK"; razoxane; sizofiran; spirogermanium; tenuazonic acid; triaziquone; 2, 2',2"'-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxanes, *e.g*. paclitaxel (TAXOL^{®}, Bristol-Myers Squibb Oncology, Princeton, NJ) and doxetaxel (TAXOTERE^{®}, Rhône-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY 117018, onapristone, and toremifene (Fareston); and anti-androgens such as flutamide, nilutamide, bicalutamide, leuprolide, and goserelin; and pharmaceutically acceptable salts, acids or derivatives of any of the above.

The term "prodrug" as used in this application refers to a precursor or derivative form of a pharmaceutically active substance that is less cytotoxic to tumor cells compared to the parent drug and is capable of being enzymatically activated or converted into the more active parent form. See, *e.g*., Wilman, "Prodrugs in Cancer Chemotherapy" Biochemical Society Transactions, 14, pp. 375-382, 615th Meeting Belfast (1986) and Stella el al., "Prodrugs: A Chemical Approach to Targeted Drug Delivery," Directed Drug Delivery, Borchardt et al., (ed.), pp. 247-267, Humana Press (1985). The prodrugs include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form include, but are not limited to, those chemotherapeutic agents described above.

A "growth-inhibitory agent" when used herein refers to a compound or composition that inhibits growth of a cell, such as an Wnt-overexpressing cancer cell, either *in vitro* or *in vivo.* Thus, the growth-inhibitory agent is one which significantly reduces the percentage of malignant cells in S phase. Examples of growth-inhibitory agents include agents that block cell cycle progression (at a place other than S phase), such as agents that induce G1 arrest and M-phase arrest. Classical M-phase blockers include the vincas (vincristine and vinblastine), taxol, maytansinoids and topo II inhibitors such as doxorubicin, daunorubicin, etoposide, and bleomycin. Those agents that arrest G 1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. Additional examples include tumor necrosis factor (TNF), an antibody capable of inhibiting or neutralizing the angiogenic activity of acidic or basic FGF or hepatocyte growth factor (HGF), an antibody capable of inhibiting or neutralizing the coagulant activities of tissue factor, protein C, or protein S (*see,* WO 91/01753, published 21 February 1991), or an antibody capable of binding to HER2 receptor (WO 89/06692), such as the 4D5 antibody (and functional equivalents thereof) (*e.g*., WO 92/22653).

"Treatment" is an intervention performed with the intention ofpreventing the development or altering the pathology of a hyper-proliferative disorder. The concept of treatment is used in the broadest sense, and specifically includes the prevention (prophylaxis), moderation, reduction, and curing of hyper-proliferative disorders of any stage. Accordingly, "treatment" refers to both therapeutic treatment and prophylactic or preventative measures, wherein the object is to prevent or slow down (lessen) or ameliorate such disorders. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial effect for an extended period of time.

"Mammal" for purposes of treatment refers to any animal classified as a mammal, including humans, domestic and farm animals, and zoo, sports, or pet animals, such as dogs, horses, cats, cows, sheep, pigs, etc. Preferably, the mammal is human.

Administration "in combination with" one or more further therapeutic agents includes simultaneous (concurrent) and consecutive administration in any order.

The term "therapeutically effective amount" refers to an amount of an antibody or a drug effective to "treat" a disease or disorder in a subject or mammal. In the case of cancer, the therapeutically effective amount of the drug may reduce the number of cancer cells; reduce the tumor size; inhibit (*i.e*., slow to some extent and preferably stop) cancer cell infiltration into peripheral organs; inhibit (*i.e*., slow to some extent and preferably stop) tumormetastasis; inhibit, to some extent, tumor growth; and/or relieve to some extent one or more of the symptoms associated with the cancer. See preceding definition of "treating". To the extent the drug may prevent growth and/or kill existing cancer cells, it may be cytostatic and/or cytotoxic.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}_{,} polyethylene glycol (PEG), and PLURONICS^{™}.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules that lack antigen specificity. Polypeptides of the latter kind are, for example, produced at low levels by the lymph system and at increased levels by myelomas. The term "antibody" is used in the broadest sense and specifically covers, without limitation, intact monoclonal antibodies, polyclonal antibodies, multispecific antibodies (*e.g*., bispecific antibodies) formed from at least two intact antibodies, and antibody fragments, so long as they exhibit the desired biological activity.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody to and for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework regions (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies. See, Kabat et al., NIH Publ. No.91-3242, Vol. I, pages 647-669 (1991). The constant domains are not involved directly in binding an antibody to an antigen, but exhibit various effector functions, such as participation of the antibody in antibody-dependent cellular toxicity.

"Antibody fragments" comprise a portion of an intact antibody, preferably the antigen-binding or variable region of the intact antibody. Examples of antibody fragments include Fab, Fab', F(ab')₂, and Fv fragments; diabodies; linear antibodies (Zapata et al., Protein Eng., 8(10): 1057-1062 (1995)); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is the minimum antibody fragment that contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH I domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM; and several of these may be further divided into subclasses (isotypes), *e.g*., IgG 1, IgG2, IgG3, IgG4, IgA1, and IgA2. The heavy-chain constant domains that correspond to the different classes of immunoglobulins are called α, δ, ε, γ, and p, respectively. The subunit structures and three-dimensional configurations of different classes of immunoglobulins are well known.

The term "monoclonal antibody" as used herein refers to an antibody obtained from a population of substantially homogeneous antibodies, *i.e*., the individual antibodies comprising the population are identical except for possible naturally-occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al., Nature, 256: 495 (1975), or may be made by recombinant DNA methods (*see, e.g.,* U.S. Patent No. 4,816,567). The "monoclonal antibodies" may also be isolated from phage antibody libraries using the techniques described in Clackson et al., Nature, 352: 624-628 (1991) and Marks et al., J. Mol. Biol., 222: 581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity. U.S. Patent No. 4,816,567; Morrison et al., Proc. Natl. Acad. Sci. USA, 81: 6851-6855 (1984).

"Humanized" forms of non-human (*e.g*., murine) antibodies are chimeric immunoglobulins, immunoglobulin chains, or fragments thereof (such as Fv, Fab, Fab', F(ab')₂, or other antigen-binding subsequences of antibodies) that contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues that are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody preferably also will comprise at least a portion of an immunoglobulin constant region (Fc), typically that of a human immunoglobulin. For further details, *see* Jones et al., Nature, 321: 522-525 (1986); Reichmann et al., Nature, 332: 323-329 (1988); and Presta, Curr. Op. Struct. Biol., 2: 593-596 (1992). The humanized antibody includes a PRIMATIZED^{™} antibody wherein the antigen-binding region of the antibody is derived from an antibody produced by immunizing macaque monkeys with the antigen of interest.

"Single-chain Fv" or "sFv" antibody fragments comprise the V_{H} and V_{L} domains of an antibody, wherein these domains are present in a single polypeptide chain. Preferably, the Fᵥ polypeptide further comprises a polypeptide linker between the V_{H} and V_{L} domains that enables the sFv to form the desired structure for antigen binding. For a review of sFv *see,* Pluckthun in The Pharmacology of Monoclonal Antibodies, Vol. 113, Rosenburg and Moore, eds. (Springer-Verlag: New York, 1994), pp. 269-315.

The term "diabodies" refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90: 6444-6448 (1993).

An "isolated" antibody is one that has been identified and separated and/or recovered from a component of its natural environment. Contaminant components of its natural environment are materials that would interfere with diagnostic or therapeutic uses for the antibody, and may include enzymes, hormones, and other proteinaceous or nonproteinaceous solutes. The antibody may be purified (1) to greater than 95% by weight of antibody as determined by the Lowry method, and most preferably more than 99% by weight, (2) to a degree sufficient to obtain at least 15 residues ofN-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells, since at least one component of the antibody's natural environment will not be present. Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The term "epitope" is used to refer to binding sites for (monoclonal or polyclonal) antibodies on protein antigens. Antibodies that bind to a certain epitope are identified by "epitope mapping." There are many methods known in the art for mapping and characterizing the location of epitopes on proteins, including solving the crystal structure of an antibody-antigen complex, competition assays, gene fragment expression assays, and synthetic peptide-based assays, as described, for example, in Chapter 11 of Harlow and Lane, Using Antibodies, a Laboratory Manual, Cold Spring Harbor Laboratory Press, Cold Spring Harbor, New York, 1999. Competition assays are discussed below. According to the gene fragment expression assays, the open reading frame encoding the protein is fragmented either randomly or by specific genetic constructions and the reactivity of the expressed fragments of the protein with the antibody to be tested is determined. The gene fragments may, for example, be produced by PCR and then transcribed and translated into protein *in vitro,* in the presence of radioactive amino acids. The binding of the antibody to the radioactively labeled protein fragments is then determined by immunoprecipitation and gel electrophoresis. Certain epitopes can also be identified by using large libraries of random peptide sequences displayed on the surface of phage particles (phage libraries). Alternatively, a defined library of overlapping peptide fragments can be tested for binding to the test antibody in simple binding assays. The latter approach is suitable to define linear epitopes of about 5 to 15 amino acids.

An antibody binds "essentially the same epitope" as a reference antibody, when the two antibodies recognize identical or sterically overlapping epitopes. The most widely used and rapid methods for determining whether two epitopes bind to identical or sterically overlapping epitopes are competition assays, which can be configured in all number of different formats, using either labeled antigen or labeled antibody. Usually, the antigen is immobilized on a 96-well plate, and the ability of unlabeled antibodies to block the binding of labeled antibodies is measured using radioactive or enzyme labels.

A "native sequence" polypeptide is one which has the same amino acid sequence as a polypeptide (e.g., antibody) derived from nature. Such native sequence polypeptides can be isolated from nature or can be produced by recombinant or synthetic means. Thus, a native sequence polypeptide can have the amino acid sequence of a naturally occurring human polypeptide, murine polypeptide, or polypeptide from any other mammalian species.

The term "amino acid sequence variant" refers to a polypeptide that has amino acid sequences that differ to some extent from a native sequence polypeptide. Ordinarily, amino acid sequence variants will possess at least about 70% homology with the native sequence, preferably, at least about 80%, more preferably at least about 85%, even more preferably at least about 90% homology, and most preferably at least 95%. The amino acid sequence variants can possess substitutions, deletions, and/or insertions at certain positions within the amino acid sequence of the native amino acid sequence.

The phrase "functional fragment or analog" of an antibody is a compound having qualitative biological activity in common with a full-length antibody. For example, a functional fragment or analog of an anti-IgE antibody is one which can bind to an IgE immunoglobulin in such a manner so as to prevent or substantially reduce the ability of such molecule from having the ability to bind to the high affinity receptor, FcεRI.

"Homology" is defined as the percentage of residues in the amino acid sequence variant that are identical after aligning the sequences and introducing gaps, if necessary, to achieve the maximum percent homology. Methods and computer programs for the alignment are well known in the art. One such computer program is "Align 2", authored by Genentech, Inc., which was filed with user documentation in the United States Copyright Office, Washington, DC 20559, on December 10, 1991.

Antibody "effector functions" refer to those biological activities attributable to the Fc region (a native sequence Fc region or amino acid sequence variant Fc region) of an antibody, and vary with the antibody isotype. The Fc region of the antibody also determines the antibody's isotype. Examples of various antibody isotypes include IgG1, IgG2, IgG3, IgG4, IgA1, IgA2, IgD, IgM and IgE. Examples of antibody effector functions include: Clq binding and complement dependent cytotoxicity (CDC); Fc receptor binding; antibody-dependent cell-mediated cytotoxicity (ADCC); phagocytosis; down regulation of cell surface receptors (*e.g*. B cell receptor); and B cell activation.

"Antibody-dependent cell-mediated cytotoxicity" or "ADCC" refers to a form of cytotoxicity in which secreted Ig bound onto Fc receptors (FcRs) present on certain cytotoxic cells (*e.g*. Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are absolutely required for such killing. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo,* e.g., in a animal model such as that disclosed in Clynes et al. PNAS (USA) 95:652-656 (1998). Moreover, techniques for modulating (*i.e*., increasing or decreasing) the level of ADCC and/or CDC activity of an antibody are well known in the art. *See, e.g.,* U.S. Patent No. 6,194,551. Antibodies preferably are incapable, or have been modified to have a reduced ability, of inducing ADCC and/or CDC.

"Fc receptor" or "FcR" describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see review M. in Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source, *e.g*. from blood.

"Complement dependent cytotoxicity" or "CDC" refers to the lysis of a target cell in the presence of complement. Activation of the classical complement pathway is initiated by the binding of the first component of the complement system (C 1 q) to antibodies (of the appropriate subclass) which are bound to their cognate antigen. To assess complement activation, a CDC assay, *e.g.* as described in Gazzano-Santoro et al., J. Immunol. Methods 202:163 (1996), may be performed.

The word "label" when used herein refers to a detectable compound or other composition that is conjugated directly or indirectly to the antibody so as to generate a "labeled" antibody. The label may be detectable by itself (*e.g*., radioisotope labels or fluorescent labels) or, in the case of an enzymatic label, may catalyze chemical alteration of a substrate compound or composition that is detectable. Radionuclides that can serve as detectable labels include, for example, I-131,I-123,I-125, Y-90, Re-188, At-211, Cu-67, Bi-212, and Pd-109. The label may also be a non-detectable entity such as a toxin.

By "solid phase" is meant a non-aqueous matrix to which an antibody can adhere. Examples of solid phases encompassed herein include those formed partially or entirely of glass (*e.g*., controlled pore glass), polysaccharides (*e.g*., agarose), polyacrylamides, polystyrene, polyvinyl alcohol and silicones. In certain embodiments, depending on the context, the solid phase can comprise the well of an assay plate; in others it is a purification column (*e.g*., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant that is useful for delivery of a drug (such as antibodies disclosed herein) to a mammal. The components of the liposome are commonly arranged in a bilayer formation, similar to the lipid arrangement of biological membranes.

As used herein, the term "immunoadhesin" designates antibody-like molecules that combine the binding specificity of a heterologous protein (an "adhesin") with the effector functions of immunoglobulin constant domains. Structurally, the immunoadhesins comprise a fusion of an amino acid sequence with the desired binding specificity that is other than the antigen recognition and binding site of an antibody (*i.e*., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG1, IgG2, IgG3, or IgG4 subtypes, IgA (including IgA1 and IgA2), IgE, IgD, or IgM.

The term "epitope tagged" used herein refers to a chimeric polypeptide comprising an antibody polypeptide fused to a "tag polypeptide". The tag polypeptide has enough residues to provide an epitope against which an antibody can be made, yet is short enough such that it does not interfere with activity of the Ig polypeptide to which it is fused. The tag polypeptide is also preferably fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

A "small molecule" is defined herein to have a molecular weight below about 500 Daltons.

The term "package insert" is used to refer to instructions customarily included in commercial packages of therapeutic products, that contain information about the indications, usage, dosage, administration, contraindications and/or warnings concerning the use of such therapeutic products.

An "isolated nucleic acid" is a nucleic acid, e.g., an RNA, DNA, or a mixed polymer, which is substantially separated from other genome DNA sequences as well as proteins or complexes such as ribosomes and polymerases, which naturally accompany a native sequence. The term embraces a nucleic acid sequence which has been removed from its naturally occurring environment, and includes recombinant or cloned DNA isolates and chemically synthesized analogues or analogues biologically synthesized by heterologous systems. A substantially pure molecule includes isolated forms of the molecule.

"Vector" includes shuttle and expression vectors. Typically, the plasmid construct will also include an origin of replication (*e.g*., the ColE1 origin of replication) and a selectable marker (*e.g*., ampicillin or tetracycline resistance), for replication and selection, respectively, of the plasmids in bacteria. An "expression vector" refers to a vector that contains the necessary control sequences or regulatory elements for expression of the antibodies including antibody fragment in bacterial or eukaryotic cells. Suitable vectors are disclosed below.

A "cancer therapy target" or "target for cancer therapy" is defined herein as a molecule, usually a polypeptide, which is capable of being bound by a heterologous molecule, has one or more binding sites for the heterologous molecule and may be the focus for designing, discovering or preparing therapeutics for the amelioration of a symptom related to cancer, or any other hyper-proliferative disease or disorder. In addition to polypeptide targets, the disclosure also encompasses non-polypeptide cancer therapy targets, such as tumor-associated glycolipid targets, as described in U.S. Patent No. 5,091,178.

An "anti-mitotic compound", as defined herein, means a compound which inhibits, prevents or delays mitotic cell division and/or progression of a cell through any stage of the cell cycle. Anti-mitotic compounds may function by affecting microtubule formation and/or action. Such agents can be, for instance, microtubule stabilizing agents or agents which disrupt microtubule formation. Microtubule affecting agents are well known to those of skill in the art and include, but are not limited to maytansine, maytansine derivatives, allocolchicine, Halichondrin B, colchicine, colchicine derivatives, dolastatin 10, rhizoxin, paclitaxel, Taxol.RTM derivatives, thiocolchicine, trityl cysteine, vinblastine sulfate, vincristine sulfate, epothilone A, epothilone, and discodermolide estramustine, nocodazole, MAP4, and the like. Examples of such agents are also described in the scientific and patent literature. *See*, *e.g*., Bulinski (1997) J. Cell Sci. 110:3055-3064; Panda (1997) Proc. Natl. Acad. Sci. USA 94:10560-10564; Muhlradt (1997) Cancer Res. 57:3344-3346; Nicolaou (1997) Nature 387:268-272; Vasquez (1997) Mol. Biol. Cell 8:973-985; Panda (1996) J. Biol. Chem. 271:29807-29812.

A "maytansinoid", as defined herein, is an ansa macrolide that is a highly toxic mitotic inhibitor which acts by inhibiting tubulin polymerization. Maytansine, which is one type of maytansinoid, was first isolated from the east African shrub *Maytenus serrata* (U.S. Patent No. 3,896,111). Maytansinoids include, but are not limited to, synthetic maytansinoid. Synthetic maytansinoid and maytansinoid analogues (*e.g*., maytansinol), are well known in the art and disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

"More highly expressed" when referring to the expression of a cell surface polypeptide, as used herein, means the copy number of the polypeptide on the surface of a cell is at least 10% higher than on another cell to which the cell is being compared, preferably 15%, 20%, 25%, 30%, 35%, 40%, 45%, 50%, 55%, 60%, 65%, 70%, 75%, 80%, 85%, 90%, 95%, 99% and most preferably 100% higher than on another cell to which the cell is being compared.

"About the same" when referring to the expression of a cell surface polypeptide, as used herein, means there is less than a 10% difference in the copy number of the polypeptide on the surface of a cell as compared to another cell, preferably less than 5%, 2%, 1% and most preferably a 0% difference in the copy number of the polypeptide on the surface of a cell as compared to another cell.

"DNA microarray" refers to an array of distinct polynucleotides or oligonucleotides arranged on a substrate such as paper, nylon or other type of membrane, filter, gel, polymer, chip, glass slide, or any other suitable support,
including solid supports. The DNA microarray is used to monitor the differential expression level of large numbers of genes simultaneously (to produce a transcript image) and to identify genetic variants, mutations and polymorphisms. Specifically, the DNA microarray is designed to detect differential or altered expression of genes derived from a first cell type, with respect to expression of the same gene in a second cell type (typically the first cell type corresponds to non-diseased tissue and the second cell type corresponds to diseased tissue). Protein arrays also are encompassed within the scope of the invention. An example of such a protein array can be found, for example, in U.S. Patent No. 6,197,599. Typically, the DNA microarray is based on the use of a database of thousands of different selected nucleic acid sequences representing gene fragments arranged on a single microscope slide by a robot. Next, the mRNA of a particular cell type (reflecting cell specific expression of a variety of genes) is converted to cDNA by RT/PCR methodology, labeled with fluorescent tags, and allowed to hybridize to the selected nucleic acid sequences on the slide. A scanner then detects and measures the fluorescence of each sample on the slide, where fluorescence represents a labeled messenger from the test cells identifiable due to its hybridization with a known nucleic acid sequence at a known position on the slide. Relative fluorescence indicates relativity activity of a gene, with strong fluorescence indicating an active gene expressing a relative large amount of messenger. Little or no fluorescence indicates that no labeled messenger hybridized to the known nucleic acid sequence.

A "proliferating" cell, as used herein, means a cell in which M phase (M=mitosis) of the cell's reproductive cycle occurs at least about every 8 hours. A "slowly proliferating" cell, as used herein, means a cell in which M phase of the cell's reproductive cycle occurs less than about every 8 hours but more than or equal to about every 72 hours. A "non-proliferating" cell, as used herein, means a cell in which M phase of the cell's reproductive cycle occurs less than about every 72 hours.

### 5.2. Compositions and Methods

### 5.2.1. Antibodies

The following describes exemplary techniques for the production of the antibodies. In some cases, the antibodies can be produced recombinantly in, and isolated from, bacterial or eukaryotic cells using standard recombinant DNA methodology.

### 5.2.1.1. Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen (especially when synthetic peptides are used) to a protein that is immunogenic in the species to be immunized. For example, the antigen can be conjugated to keyhole limpet hemocyanin (KLH), serum albumin, bovine thyroglobulin, or soybean trypsin inhibitor, using a bifunctional or derivatizing agent, e.g., maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, *e.g*., 100 µg or 5 µg of the protein or conjugate (for rabbits or mice, respectively) with 3 volumes of Freund's complete adjuvant and injecting the solution intradermally at multiple sites. One month later, the animals are boosted with 1/5 to 1/10 the original amount of peptide or conjugate in Freund's complete adjuvant by subcutaneous injection at multiple sites. Seven to 14 days later, the animals are bled and the serum is assayed for antibody titer. Animals are boosted until the titer plateaus. Conjugates also can be made in recombinant cell culture as protein fusions. Also, aggregating agents such as alum are suitably used to enhance the immune response.

### 5.2.1.2. Monoclonal Antibodies

Monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium which medium preferably contains one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

Preferred fusion partner myeloma cells are those that fuse efficiently, support stable high-level production of antibody by the selected antibody-producing cells, and are sensitive to a selective medium that selects against the unfused parental cells. Preferred myeloma cell lines are murine myeloma lines, such as those derived from MOPC-21 and MPC-11 mouse tumors available from the Salk Institute Cell Distribution Center, San Diego, California USA, and SP-2 and derivatives e.g., X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980).

Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-M EM or RPMI-1640 medium. In addition, the hybridoma cells may be grown *in vivo* as ascites tumors in an animal e.g, by i.p. injection of the cells into mice.

The monoclonal antibodies secreted by the subclones are suitably separated from the culture medium, ascites fluid, or serum by conventional antibody purification procedures such as, for example, affinity chromatography (e.g., using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (*e.g*., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs., 130:151-188 (1992).

Monoclonal antibodies orantibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain (C_{H} and C_{L}) sequences for the homologous murine sequences (U.S. Patent No. 4,816,567; and Morrison, et al., Proc. Natl Acad Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### 5.2.1.3. Humanized Antibodies

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Reichmann et al., Nature, 332:323-327 (1988); Verhoeyen et al., Science, 239:1534-1536 (1988)), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol., 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high binding affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e*., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgGl antibody.

### 5.2.1.4. Human Antibodies

As an alternative to humanization, human antibodies can be generated. For example, it is now possible to produce transgenic animals (*e.g*., mice) that are capable, upon immunization, ofproducing a full repertoire of human antibodies in the absence of endogenous immunoglobulin production. For example, it has been described that the homozygous deletion of the antibody heavy-chain joining region (J_{H}) gene in chimeric and germ-line mutant mice results incomplete inhibition of endogenous antibody production. Transfer of the human germ-line immunoglobulin gene array into such germ-line mutant mice will result in the production of human antibodies upon antigen challenge. See, *e.g*., Jakobovits et al., Proc. Natl. Acad. Sci. USA, 90:2551 (1993); Jakobovits et al., Nature, 362:255-258 (1993); Bruggemann et al., Year in Immuno., 7:33 (1993); U.S. Patent Nos. 5,545,806, 5,569,825, 5,591,669 (all of GenPharm); 5,545,807; and WO 97/17852.

Alternatively, phage display technology (McCafferty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M 13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, *e.g*., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patents 5,567,610 and 5,229,275).

### 5.2.1.5. Antibody Fragments

In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, *e.g*., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from *E. coli,* thus allowing the facile production of large amounts of these fragments. Antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10: 163-167 (1992)). According to another approach, F(ab')₂ fragments can be isolated directly from recombinant host cell culture. Fab and F(ab')₂ fragment with increased in vivo half-life comprising a salvage receptor binding epitope residues are described in U.S. Patent No. 5,869,046. Other techniques for the production of antibody fragments will be apparent to the skilled practitioner. The antibody of choice may be a single chain Fv fragment (scFv). See WO 93/16185; U.S. Patent No. 5,571,894; and U.S. Patent No. 5,587,458. Fv and sFv are the only species with intact combining sites that are devoid ofconstant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", *e.g.,* as described in U.S. Patent 5,641,870 for example. Such linear antibody fragments may be monospecific or bispecific.

### 5.2.1.6. Bispecific Antibodies

Bispecific antibodies are antibodies that have binding specificities for at least two different epitopes. Exemplary bispecific antibodies may bind to two different epitopes of an antigen. Other such antibodies may combine an antigen binding site with a binding site for another protein. Bispecific antibodies may also be used to localize cytotoxic agents to cells which express a specific antigen. These antibodies possess an antigen-binding arm and an arm which binds the cytotoxic agent (*e.g*. saporin, anti-interferon-α, vinca alkaloid, ricin A chain, methotrexate or radioactive isotope hapten). Bispecific antibodies can be prepared as full length antibodies or antibody fragments (*e.g*. F(ab')₂ bispecific antibodies).

Methods for making bispecific antibodies are known in the art. Traditional production of full length bispecific antibodies is based on the co-expression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

According to a different approach, antibody variable domains with the desired binding specificities (antibody-antigen combining sites) are fused to immunoglobulin constant domain sequences. Preferably, the fusion is with an Ig heavy chain constant domain, comprising at least part of the hinge, C_{H}2, and C_{H}3 regions. It is preferred to have the first heavy-chain constant region (C_{H}1) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination.

In a preferred embodiment of this approach, the bispecifc antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the C_{H}3 domain. In this method, one or more small amino acid side chains from the interface of the first antibody molecule are replaced with larger side chains (*e.g*. tyrosine or tryptophan). Compensatory "cavities" of identical or similar size to the large side chain(s) are created on the interface of the second antibody molecule by replacing large amino acid side chains with smaller ones (*e.g*. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')₂ fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli*, which can be chemically coupled to form bispecific antibodies. Shalaby et al., J. Exp. Med., 175: 217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipperpeptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a V_{H} connected to a V_{L} by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### 5.2.1.7. Multivalent Antibodies

A multivalent antibody may be internalized (and/or catabolized) faster than a bivalent antibody by a cell expressing an antigen to which the antibodies bind. The antibodies can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-(X1)ₙ-VD2-(X2)ₙ-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 5.2.2. Amino Acid Modifications

Amino acid sequence modification(s) of the antibodies described herein are contemplated. For example, it may be desirable to improve the binding affinity and/or other biological properties of the antibody. Amino acid sequence variants of the antibodies are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics. The amino acid changes also may alter post-translational processes of the antibody, such as changing the number or position of glycosylation sites.

A useful method for identification ofcertain residues or regions of the antibody that are preferred locations for mutagenesis is called "alanine scanning mutagenesis" as described by Cunningham and Wells in Science, 244:1081-1085 (1989). Here, a residue or group of target residues are identified (*e.g*., charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, ala scanning or random mutagenesis is conducted at the target codon or region and the expressed antibody variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme (*e.g*. for ADEPT) or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened.

**TABLE 1: Amino Acid Substitutions**

| **Original Residue** | **Exemplary Substitutions** | **Preferred Substitutions** |
|---|---|---|
| Ala (A) | val; leu; ile | val |
| Arg (R) | lys; gln; asn | lys |
| Asn (N) | gln; his; asp, lys; arg | gln |
| Asp (D) | glu; asn | glu |
| Cys (C) | ser; ala | ser |
| Gin (Q) | asn; glu | asn |
| Glu (E) | asp; gln | asp |
| Gly(G) | ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | tyr |
| Pro (P) | ala | ala |
| Ser (S) | thr | thr |
| Thr(T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other techniques known in the art.

Scanning amino acid analysis can also be employed to identify one ormore amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

### 5.2.2.1. Additional Modifications

A particularly preferred type of substitutional variant involves substituting one or more hypervariable region residues of a parent antibody (*e.g*. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (*e.g*. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (*e.g*. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody alters the original glycosylation pattern of the antibody. By altering is meant deleting one or more carbohydrate moieties found in the antibody, and/or adding one or more glycosylation sites that are not present in the antibody. In addition, the phrase includes qualitative changes in the glycosylation pattern of the antibody, involving a change in the nature and proportions of various carbohydrate moieties present.

Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

Another means of increasing the number of carbohydrate moieties on the antibodies is by chemical or enzymatic coupling of glycosides to the antibody. Such methods are described in the art, *e.g*., in WO 87/05330 published 11 September 1987, and in Aplin and Wriston, CRC Crit. Rev. Biochem., pp. 259-306 (1981).

Removal of carbohydrate moieties present on the antibody may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of the antibody comprises linking the antibody to one of a variety of nonproteinaceous polymers, *e.g*., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The antibody may also be modified in a way to form a chimeric molecule comprising the antibody fused to another, heterologous polypeptide or amino acid sequence.

In one embodiment, such a chimeric molecule comprises a fusion of the antibody with a tag polypeptide which provides an epitope to which an anti-tag antibody can selectively bind. The epitope tag is generally placed at the amino- or carboxyl- terminus of the antibody. The presence of such epitope-tagged forms of the antibody can be detected using an antibody against the tag polypeptide. Also, provision of the epitope tag enables the antibody to be readily purified by affinity purification using an anti-tag antibody or another type of affinity matrix that binds to the epitope tag. Various tag polypeptides and their respective antibodies are well known in the art. Examples include poly-histidine (poly-His) or poly-histidine-glycine (poly-His-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553(1990)]. Other tag polypeptides include the Flag-peptide [Hopp et al., BioTechnology, 6:1204-1210(1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

### 5.2.3. Effector Function Engineering

It may be desirable to modify the antibody with respect to effector function, *e.g*. so as to enhance or inhibit antigen-dependent cell-mediated cyotoxicity (ADCC) and/or complement dependentcytotoxicity (CDC) of the antibody. This may be achieved by introducing one or more amino acid substitutions in an Fc region of the antibody. *See, e.g.,* U.S. Patent No. 6,194,551. Alternatively or additionally, cysteine residue(s) may be introduced in the Fc region, thereby allowing interchain disulfide bond formation in this region. The homodimeric antibody thus generated may have improved or reduced internalization capability and/or increased or decreased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992).

Homodimeric antibodies with enhanced anti-tumor activity may also be prepared using heterobifunctional cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al. Anti-Cancer Drug Design 3:219-230 (1989).

The antibodies preferably have a reduced ability to induce ADCC or CDC. As noted above, methods of modifying antibodies to yield antibodies with a reduced ability to induce ADCC and/or CDC are well known in the art. *See, e.g.,* U.S. Patent No. 6,194,551. Since the antibodies of the invention preferably are conjugated to anti-mitotic compounds to produce cytotoxic compounds, by reducing and/or eliminating the ADCC and/or CDC capacity of the antibodies, the therapeutic effect (*e.g*., cell killing of only proliferating cells) of the cytotoxic compound will be mediated primarily by the anti-mitotic component of the cytotoxic compound, rather than by indirect cell killing via ADCC and/or CDC.

To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (*e.g*., IgG₁, IgG₂, IgG₃, or IgG₄) that is responsible for increasing the *in vivo* serum half-life of the IgG molecule.

### 5.2.4. Immunoconjugates

Immunoconjugates comprising an antibody conjugated to a cytotoxic agent such as a chemotherapeutic agent, toxin (*e.g*., an enzymatically active toxin of bacterial, fungal, plant, or animal origin, or fragments thereof), a radioactive isotope (*i.e*., a radioconjugate), a growth inhibitory agent or an anti-mitotic compound.

### 5.2.4.1. Maytansinoid Immunoconjugates

An antibody may be conjugated to one or more maytansinoid molecules without significantly diminishing the biological activity of either the antibody or the maytansinoid molecule(s). Maytansinoids are mitotic inhibitors which act by inhibiting tubulin polymerization. Thus, the immunoconjugates with maytansinoids are therapeutically effective against cells which are proliferating (*e.g*., cancer cells), and thus, undergoing mitosis. The same immunoconjugates, however, would not be effective against cells which are not proliferating.

The maytansinoid immunoconjugates would be targeted to tissues and/or cells which do not undergo maytansinoid-induced toxicity. Examples of tissues/cells which have previously been demonstrated to undergo maytansinoid-induced toxicity include, but are not limited to, gastrointestinal (GI) tissues and neuronal cells (Issel et al., 1978, Can. Trimnt. Rev., 5:199-207). The GI system likely exhibits toxicity in response to maytansinoids because the cells within the GI are highly proliferating and, thus, the anti-mitotic properties of the maytansinoids would tend to kill such cells, Although neuronal cells are not highly proliferating cells, neurons depend upon microtubules for axonal vesicular transport. As noted previously, maytansinoids are mitotic inhibitors because they inhibit tubulin polymerization. Thus, the inhibition of tubulin polymerization by maytansinoids could adversely affect neuronal cells, even though neurons are not highly proliferating.

Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and various maytansinol esters.

There are many linking groups known in the art for making antibody-maytansinoid conjugates, including, for example, those disclosed in U.S. Patent No. 3.208,020 or EP Patent 0 425 235 B1, and Chari et al. Cancer Research 52: 127-131 (1992). The linking groups include disulfide groups, thioether groups, said labile groups, photolabile groups, peptidase labile groups, or esterase labile groups, as disclosed in the above-identified patents, disulfide and thioether groups being referred.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters) (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzone). Particularly preferred coupling agents include N-succinimidyl-3-(2-pyridyldithio) propionate (SPDP) (Carisson et al., Biochem. J. 173:723-737 [1978]) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hyrdoxymethyl, the C-15 position modified with a hydroxyl group, and the C-20 position having a hydroxyl group. The linkage may be formed at the C-3 position of maytansinol or a maytansinol analogue.

### 5.2.4.2 Other Immungoconjugates

Other antitumor agents that can be conjugated to the antibodies include BCNU, streptozoicin, vincristine and 5-fluorouracil, the family of agents known collectivelyLL-E33288 complex described in U.S. patents 5,053,394, 5,770,710, as well as esperamicins (U.S. patient 5,877,296).

Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes, See, for example, WO 93/21232 published October 28, 1993.

The description further contemplates an immunoconjugate formed between an antibody and a compound with nucleolytic activity (*e.g*. a ribonuclease or a DNA endonuclease such as a deoxyribonuclease; DNase).

For selective destruction of the tumor, the antibody may comprise a highly radioactive atom. A variety of radioactive isotopes are available for the production of radioconjugated antibodies. Examples include At²¹¹, 1¹³¹, 1¹²⁵, Y⁹⁰, Re¹⁸⁶, Re ¹⁸⁸, Sm¹⁵³, Bi²¹², P³², Pb²¹² and radioactive isotopes of Lu. When the conjugate is used for diagnosis, it may comprise a radioactive atom for scintigraphic studies, for example tc^{99m} or I¹²³, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemicalamino acid synthesis using suitable amino acid precursors involving, for example, fluorine-19 in place of hydrogen. Labels such as tc^{99m} or I¹²³, Re¹⁸⁶, Re¹⁸⁸ and In¹¹¹ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothlolane (IT), bifunctional derivatives of imidoesters (such as dimethyl adipimidate HCL), active eaters (such as disuccinimidyl suberate), aldehydes (suchas glutareldehyde), bis-azido compounds (such as bis (p-azidabonzoyl) hexanediamine), bis-diazonium derivatives (such as bis-(p-diazoniumbenzoyl)-athylenediamine), diisocyanates (such as tolyene 2,6-diisocyanate), and bis-active fluorine compounds (such as 1,3-difluoro-2,4-dinitrobenzene). For example, a ricin immunotoxin con be prepared as described in Vitetta at al. Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al. Cancer Research 52; 127-131 (1992); U.S. Patent No. 5,208,020) may be used.

Alternatively, a fusion protein comprising the antibody and cytotoxic agent may be made, *e.g*. by recombinant techniques or peptide synthesis. The length of DNA may comprise respective regions encoding the two portions of the conjugate either adjacent one another or separated by a region encoding a linker peptide which does not destroy the desired properties of the conjugate.

The antibody may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a "ligand" (*e.g*. avidin) which is conjugated to a cytotoxic agent (*e.g*. a radionucleotide).

### 5.2.5. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl. Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody can be conjugated to the liposomes as described in Martin et al., J. Biol. Chem., 257: 286-288 (1982) via a disulfide-interchange reaction. A chemotherapeutic agent (such as Doxorubicin) is optionally contained within the liposome. *See*, Gabizon et al.,J. National Cancer Inst., 81(19): 1484 (1989).

### 5.2.6. Administration Protocols, Schedules, Doses, and Formulations

The compounds herein are pharmaceutically useful as a prophylactic and therapeutic agent for various disorders and diseases as set forth above.

Therapeutic compositions of the compounds are prepared for storage by mixing the desired compounds having the appropriate degree of purity with optional pharmaceutically acceptable carriers, excipients, or stabilizers (Remington's Pharmaceutical Sciences, 16th edition, Osol, A. ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10. residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol , trehalose or sorbitol; salt-forming counter-ions such as sodium; metal complexes (*e.g*., Zn-protein complexes); and/or non-ionio surfactants such as TWEEN^{™}, PLURONICS^{™} or polyethylene glycol (PEG).

Additional examples of such carriers include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and polyethylene glycol. Carriers for topical or gel-based forms of the compounds include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. For all administrations, conventional depot forms are suitably used. Such forms include, for example, microcapsules, nano-capsulea, liposomes, plasters, inhalation forms, nose sprays, sublingual tablets, and sustained-release preparations, The compounds will typically be formulated in such vehicles at a concentration of about 0.1 mg/ml to 100 mg/ml.

Compounds to be used for *in vivo* administration must be sterile. This is readily accomplished by filtration through sterile filtration membranes, prior to or following lyophilization and reconstitution. Compounds ordinarily will be stored in lyophilized form or in solution if administered systemically. If in lyophilized form, the compound is typically formulated in combination with other ingredients for reconstitution with an appropriate diluent at the time for use. An example of a liquid formulation of a compound is a sterile, clear, colorless unpreserved solution filled in a single-dose vial for subcutaneous injection. Preserved pharmaceutical compositions suitable for repeated use may contain, for example, depending mainly on the indication and type of polypeptide:
a) a compound;
b) a buffer capable of maintaining the pH in a range of maximum stability of the polypeptide or other molecule in solution, preferably about 4-8;
c) a detergent/surfactant primarily to stabilize the compound against agitation-induced aggregation;
d) an isotonifier;
e) a preservative selected from the group of phenol, benzyl alcohol and a benzethonium halide, *e.g*., chloride; and
f) water.

If the detergent employed is non-ionic, it may, for example, be polysorbates (*e.g*., POLYSORBATE^{™} (TWEEN^{™}) 20, 80, etc.) or poloxamers (*e.g*., POLOXAMER^{™} 188). The use of non-ionic surfactants permits the formulation to be exposed to shear surface stresses without causing denaturation of the polypeptide. Further, such surfactant-containing formulations may be employed in aerosol devices such as those used in a pulmonary dosing, and needleless jet injector guns (*see*, *e.g.,* EP 257,956).

An isotonifier may be present to ensure isotonicity of a liquid composition of the compound, and includes polyhydric sugaralcohols, preferably trihydric or higher sugar alcohols, such as glycerin, erythritol, arabitol, xylitol, sorbitol, and mannitol. These sugar alcohols can be used alone or in combination. Alternatively, sodium chloride or other appropriate inorganic salts may be used to render the solutions isotonic.

The buffer may, for example, be an acetate, citrate, succinate, or phosphate buffer depending on the pH desired. The pH of one type of liquid formulation is buffered in the range of about 4 to 8, preferably about physiological pH.

The preservatives phenol, benzyl alcohol and benzethonium halides, *e.g*., chloride, are known antimicrobial agents that may be employed.

Therapeutic compositions generally are placed into a container having a sterile access port, for example, an intravenous solution bag or vial having a stopper pierceable by a hypodermic injection needle. The formulations are preferably administered as repeated intravenous (i.v.), subcutaneous (s.c.), or intramuscular (i.m.) injections, or as aerosol formulations suitable for intranasal or intrapulmonary delivery (for intrapulmonary delivery *see, e.g.,* EP 257,956).

The compositions can also be administered in the form of sustained-released preparations. Suitable examples of sustained-release preparations include semipermeable matrices of solid hydrophobic polymers containing the compounds which matrices are in the form of shaped articles, *e.g*., films, or microcapsules. Examples of sustained-release matrices include polyesters, hydrogels (*e.g*., poly(2-hydroxyethylmethacrylate) as described by Langer et al., J. Biomed. Mater. Res.,15: 167-277 (1981) and Langer, Chem. Tech., 12: 98-105 (1982) or poly(vinylalcohol)), polylactides (U.S. Patent No. 3,773,919, EP 58,481), copolymers of L-glutamic acid and gamma ethyl-L-glutamate (Sidman et al., Biopolymers, 22: 547-556 (1983)), non-degradable ethylene-vinyl acetate (Langer *et al., supra*), degradable lactic acid-glycolic acid copolymers such as the Lupron Depot^{™} (injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C, resulting in a loss of biological activity and possible changes in immunogenicity. Rational strategies can be devised for protein stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Sustained-release compositions also include liposomally entrapped compounds. Liposomes containing the compounds are prepared by methods known *per se*: DE 3,218,121; Epstein et al., Proc. Natl. Acad. Sci. USA, 82: 3688-3692 (1985); Hwang et al., Proc. Natl. Acad. Sci. USA, 77: 4030-4034 (1980); EP 52,322; EP 36,676; EP 88,046; RP 143,949; BP 142,641; Japanese patent application 83-118008; U.S. Patent Nos. 4,485,045 and 4,544,545; and EP 102,324. Ordinarily the liposomes are of the small (about 200-800 Angstroms) unilamellar type in which the lipid content is greater than about 30 mol % cholesterol, the selected proportion being adjusted for the optimal therapy.

The therapeutically effective dose of a compound will, of course, vary depending on such factors as the pathological condition to be treated (including prevention), the method of administration, the type of compound being used for treatment, any co-therapy involved, the patient's age, weight, general medical condition, medical history, etc., and its determination is well within the skill of a practicing physician. Accordingly, it will be necessary for the therapist to titer the dosage and modify the route of administration as required to obtain the maximal therapeutic effect. The clinician will administer the compound until a dosage is reached that achieves the desired effect for treatment of the condition in question.

With the above guidelines, the effective dose generally is within the range of from about 0.001 to about 1.0 mg/kg, more preferably about 0.01-1.0 mg/kg, most preferably about 0.01-0.1 mg/kg.

The route of administration of the compounds is in accord with known methods, *e.g*., by injection or infusion by intravenous, intramuscular, intracerebral, intraperitoneal, intracerobrospinal, subcutaneous, intraocular, intraarticular, intrasynovial, intrathecal, oral, topical, or inhalation routes, or by sustained-release systems as noted below. The compounds also are suitably administered by intratumoral, peritumoral, intralesional, or perilesional routes, to exert local as well as systemic therapeutic effects. The intraperitoneal route is expected to be particularly useful, for example, in the treatment of ovarian tumors.

Examples of pharmacologically acceptable salts of molecules that form salts and are useful hereunder include alkali metal salts (*e.g*., sodium salt, potassium salt), alkaline earth metal salts (*e.g*., calcium salt, magnesium salt), ammonium salts, organic base salts (*e.g*., pyridine salt, triethylamine salt), inorganic acid salts (*e.g*., hydrochloride, sulfate, nitrate), and salts of organic acid (*e.g*., acetate, oxalate, p-toluenesulfonate).

### 5.2.7. Combination Therapies

The effectiveness of the compounds in preventing or treating the disorder in question may be improved by administering the active agent serially or in combination with another agent that is effective for those purposes, either in the same composition or as separate compositions.

The compounds when used to treat cancer may be combined with cytotoxic, chemotherapeutic, or growth-inhibitory agents as identified above. Also, for cancer treatment, the compound is suitably administered serially or in combination with radiological treatments, whether involving irradiation or administration of radioactive substances.

The effective amounts of the therapeutic agents administered in combination with the compounds will be at the physician's or veterinarian's discretion. Dosage administration and adjustment is done to achieve maximal management of the conditions to be treated. The dose will additionally depend on such factors as the type of the therapeutic agent to be used and the specific patient being treated.

### 5.2.8. Articles of Manufacture

An article of manufacture such as a kit containing the compounds useful for the treatment of the disorders described above comprises at least a container and a label. Suitable containers include, for example, bottles, vials, syringes, and test tubes. The containers may be formed from a variety of materials such as glass or plastic. The container holds a composition that is effective for diagnosing or treating the condition and may have a sterile access port (for example, the container may be an intravenous solution bag or a vial having a stopper pierceable by a hypodermic injection needle). The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use. The article of manufacture may also comprise a second or third container with another active agent as described above.

### 5.2.9. Pharmaceutical Compositions of Antibodies

Therapeutic formulations of the antibodies and immunoconjugates used are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as acetate, Tris, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; tonicifiers such as trehalose and sodium chloride; sugars such as sucrose, mannitol, trehalose or sorbitol; surfactant such as polysorbate; salt-forming counter-ions such as sodium; metal complexes (*e.g*. Zn-protein complexes); and/or non-ionic surfactants such as TWEEN^{™}, PLURONICS^{™} or polyethylene glycol (PEG). The antibody preferably comprises the antibody at a concentration ofbetween 5-200 mg/ml, preferably between 10-100 mg/ml.

If the antigen is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. *See, e.g.,* Marasco et al., Proc. Natl. Acad. Sci. USA. 90: 7889-7893 (1993).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, *supra*.

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g*., films, or microcapsules. Examples of sustained-release matrices include polyesters, hydrogels (for example, poly(2-hydroxyethyl-methacrylate), or poly(vinylaleohol)), polylactides (U.S. Pat. No, 3,773,919), copolymers of L-glutamic acid and γ ethyl-L-glutamate, non-degradable ethylene-vinyl acetate, dogradab le lactic acid-glycolic acid copolymers such as the LUPRONDEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods, When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### 5.2.10. Methods of Treatment using the Antibody

It is contemplated that the antibodies, and in particular the immunoconjugates (*e.g.*, maytansinoid-antibody conjugates), may be used to treat various diseases and disorders as noted above.

The antibodies are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred.

Generally, the disease or disorder to be treated is cancer. Examples of cancer to be treated include, but are not limited to, carcinoma, lymphoma, blastoma, sarcoma, and leukemia or lymphoid malignancies. More particular examples of such cancers include squamous cell carcinoma, (e.g., epithelial squamous cell carcinoma), lung cancer including small-call lung cancer, non-small cell lung cancer, adenocarcinoma of the lung and squamous carcinoma of the lung, cancer of the peritoneum, bepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

Other therapeutic regimens may be combined with the administration of the antibodies as noted above. For example, if the antibodies are to treat cancer, the patient to be treated with such antibodies may also receive radiation therapy. Alternatively, or in addition, a chemotherapeutic agent may be administered to the patient. Preparation and dosing schedules for such chemotherapeutic agents may be used according to manufacturers instructions or as determined empirically by the skilled practitioner. Preparation and dosing schedules for such chemotherapy are also described in Chemotherapy Service, Ed., M.C. Perry (Williams & Wilkins; Baltimore, MD, 1992). The chemotherapeutic agent may precede, or follow administration of the antibody, or may be given simultaneously therewith. The antibody may be combined with an anti-estrogen compound such as tamoxifen or EVISTA^{™} or an anti-progesterone such as onapristone (see, EP 616812) in dosages known for such molecules.

The antibody therapeutic treatment method may involve the combined administration of an antibody (or antibodies) and one or more chemotherapeutic agents or growth inhibitory agents, including co-administration of cocktails of different chemotherapeutic agents. Chemotherapeutic agents include estramustine phosphate, prednimustine, cisplatin, 5-fluorouracil, melphalan, cyclophosphamide, hydroxyurea and hydroxyureataxanes (such as paclitaxel and doxetaxel) and/or anthracycline antibiotics. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Porry, Williams & Wilkins, Baltimore, MD (1992).

The antibody may be combined with an anti-hormonal compound; e.g., an anti-estrogen compound such as tamoxifen; an anti-progesterone such as onapristone (see, EP 616 812); or an anti-androgen such as flutamide, in dosages known for such molecules. Where the cancer to be treated is androgen independent cancer, the patient may previously have been subjected to anti-androgen therapy and, after the cancer becomes androgen independent, the antibody (and optionally other agents as described herein) may be administered to the patient.

Sometimes, it may be beneficial to also co-administer a cardioprotectant (to prevent or reduce myocardial dysfunction associated with the therapy) or one or more cytokines to the patient. In addition to the above therapeutic regimes, the patient may be subjected to surgical removal of cancer cells and/or radiation therapy, before, simultaneously with, or post antibody therapy. Suitable dosages for any of the above co-administered agents are those presently used and may be lowered due to the combined action (synergy) of the agent and antibody.

If the antibodies are used for treating cancer, it may be desirable also to administer antibodies against other tumor-associated antigens, such as antibodies that bind to one or more of the ErbB2, EGFR, ErbB3, ErbB4, or VEGF receptor(s). These also include the agents set forth above. Also, the antibody is suitably administered serially or in combination with radiological treatments, whether involving irradiation or administration of radioactive substances. Alternatively, or in addition, two or more antibodies binding the same or two or more different antigens disclosed herein may be co-administered to the patient. Sometimes, it may be beneficial also to administer one or more cytokines to the patient. The antibodies herein may be co-administered with a growth-inhibitory agent. For example, the growth-inhibitory agent may be administered first, followed by an antibody. However, simultaneous administration or administration of the antibody first is also contemplated.

Vascularization of tumors may be attacked in combination therapy. The antibody and another known antibody (*e.g*., anti-VEGF) are administered to tumor-bearing patients at therapeutically effective doses as determined, for example, by observing necrosis of the tumor or its metastatic foci, if any. This therapy is continued until such time as no further beneficial effect is observed or clinical examination shows no trace of the tumor or any metastatic foci. Then TNF is administered, alone or in combination with an auxiliary agent such as alpha-, beta-, or gamma-interferon, anti-HER2 antibody, heregulin, anti-heregulin antibody, D-factor, interleukin-1 (IL-1), interleukin-2 (IL-2), granulocyte-macrophage colony stimulating factor (GM-CSF), or agents that promote microvascular coagulation in tumors, such as anti-protein C antibody, anti-protein S antibody, or C4b binding protein (*see,* WO 91/01753, published 21 February 1991), or heat or radiation.

Since the auxiliary agents will vary in their effectiveness, it is desirable to compare their impact on the tumor by matrix screening in conventional fashion. The administration of the antibody and TNF is repeated until the desired clinical effect is achieved. Alternatively, the antibody is administered together with TNF and, optionally, auxiliary agent(s). In instances where solid tumors are found in the limbs or in other locations susceptible to isolation from the general circulation, the therapeutic agents described herein are administered to the isolated tumor or organ. AFGF or PDGF antagonist, such as an anti-FGF or an anti-PDGF neutralizing antibody, may be administered to the patient in conjunction with the antibody. Treatment with antibodies preferably may be suspended during periods of wound healing or desirable neovascularization.

For the prevention or treatment of disease, the dosage and mode of administration will be chosen by the physician according to known criteria. The appropriate dosage of antibody will depend on the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Preferably, the antibody is administered by intravenous infusion or by subcutaneous injections. Depending on the type and severity of the disease, about 1 µg/kg to about 50 mg/kg body weight (*e.g*. about 0.1-15mg/kg/dose) of antibody can be an initial candidate dosage for administration to the patient, whether, for example, by one or more separate administrations, or by continuous Infusion. A dosing regimen can comprise administering an initial loading dose of about 4 mg/kg, followed by a weekly maintenance dose of about 2 mg/kg of the antibody, However, other dosage regiments may be useful. A typical daily dosage might range from about 1µg/kg to 100 mg/kg or more, depending on the factors mentioned above. For repeated administrations over several days or longer, depending on the condition, the treatment is sustained until a desired suppression of disease symptoms occurs. The progress of this therapy can be readily monitored by conventional methods and assays and based on criteria known to the physician or other persons of skill in the art,

Aside from administration of the antibody protein to the patient, the present application contemplates administration of the antibody by gene therapy. Such administration of nucleic acid encoding the antibody is encompassed by the expression "administering a therapeutically effective amount of an antibody". See, for example, WO96/0732 published March 14, 1996 concerning the use of gene therapy to generate intracellular antibodies.

There are two major approaches to getting the nucleic acid (optionally contained in a vector) into the patient's cells; *in vivo* and *ex vivo*. For *in vivo* delivery the nucleic acid is injected directly into the patient, usually at the site where the antibody is required. For ex *vivo* treatment, the patient's cells are removed, the nucleic acid is introduced into these isolated cells and the modified cells are administered to the patient either directly or, for example, encapsulated within porous membranes which are implanted into the patient (see, *e.g*. U.S. Patent Nos, 4,892,538 and 5,283,187). There are a variety of techniques available for introducing nucleic acids into viable cells. The techniques vary depending upon whether the nucleic acid is transferred into cultured cells *in vitro,* or in *vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, *etc.* A commonly used vector for ex *vivo* delivery of the gene is a retroviral vector.

The currently preferred *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). For review of the currently know gene marking and gene therapy protocols see Anderson et al., Science 256:808-813 (1992). See also WO 93/25673 and the references cited therein.

### 5.2.10.1. Antibody Targeting

It is contemplated that the antibodies may be conjugated to anti-mitotic compounds, including, but not limited to maytansinoids. Mitosis is a cellular process involved in cellular division and replication. Unfortunately, many diseases and disorders are characterized by aberrant cell mitosis. Cancer, for example, is one such example wherein cell mitosis becomes uncontrolled. Thus, it is desirable to target therapeutic agents to cells involved in aberrant cell mitosis.

The present invention is based on the discovery that antibody-anti-mitotic conjugates are highly effective at killing cells undergoing mitosis. Moreover, the invention is based on the surprising discovery that the antibody, or other cell binding agent, does not need to be specific for polypeptide antigens expressed only on tumor cells. Rather, the antibody or other cell binding agent need only differentiate between polypeptide antigens which are more highly expressed on proliferating cancer cells as compared to proliferating non-cancer cells. Since the antibody or other cell binding agent is conjugated to an anti-mitotic agent, binding of the antibody to a non-proliferating or slowly proliferating cell will not lead to death of the non-proliferating or slowly proliferating cell because only cells undergoing mitosis (*i.e*., proliferating cells) will be killed.

Wherein the antibody is conjugated to a maytansinoid, the antibody preferably would be targeted to tissues and/or cells which do not undergo maytansinoid-induced toxicity. Examples of tissues which have previously been demonstrated to undergo maytansinoid-induced toxicity include, but are not limited to, gastrointestinal (GI) tissues and neuronal cells (Issel et al., 1978, Can. Trtmnt. Rev., 5:199-207). The GI system likely exhibits toxicity in response to maytansinoids because the cells within the GI are highly proliferating and, thus, the anti-mitotic properties of the maytansinoids would tend to kill such cells. Although neuronal cells are not highly proliferating cells, neurons depend upon microtubules for axonal vesicular transport. As noted previously, maytansinoids are mitotic inhibitors because they inhibit tubulin polymerization. Thus, the inhibition of tubulin polymerization by maytansinoids could adversely affect neuronal cells, even though neurons are not highly proliferating.

Thus, the present invention overcomes limitations and deficiencies of prior tumor antigen screening methods. More specifically, prior to the instant invention, polypeptide antigens found on the surface of both tumor cells and on the surface of normal, non-proliferating or slowly proliferating cells, would not have been pursued as cancer therapy targets for the fear of toxic side effects on the non-proliferating or slowly proliferating cells. As noted above, however, the antibodies of the present invention are conjugated to anti-mitotic compounds (*e.g*., maytansinoids) and, thus, will not adversely effect non-proliferating or slowly proliferating cells. Therefore, the surprising discovery of the instant invention significantly expands the scope ofpotential polypeptide antigen targets which can be used as cancer therapeutics.

### 5.2.11. Cell Surface Polypeptide Screening Techniques

Identification of differential expression and/or altered expression of cell surface polypeptides in proliferating cancer cells and other diseased tissues as compared with proliferating, slowly proliferating and non-proliferating non-cancer cells and tissues will give valuable insights to gene function, the genetic basis of disease and therapeutic targets for the treatment of such diseases. Numerous techniques currently exist which can be used for the identification of polypeptide antigens that are more highly expressed on one cell type versus another cell type. In a preferred aspect, these techniques can be used to identify polypeptide antigens which are more highly expressed on the surface of proliferating cancer cells than on the surface of proliferating non-cancer cells. Examples of such techniques include, but are not limited to, microarray analysis, Northerns, Westerns, PCR-based strategies, TAQMAN, gene amplification and screening of gene expression databases, including, for example, public (*e.g*., Genbank) and/or private (LIFESEQ^{®}, Incyte Pharmaceuticals, Inc., Palo Alto, CA; and GeneEXPRESS^{®}, GeneLogic, Inc., Gaithersberg, MD) gene expression databases.

### 5.2.11.1. Microarray Analysis

In the past several years, a new technology called DNA microarray has gained widespread interest among molecular biologists and has been used to profile complex diseases and discover novel disease-related genes. (Ekins, R., et al., "Microarrays: their origins and applications", Trends in Biotechnology, 17: 217-218 (1999); Heller, A. et al., Proc. Natl. Acad. Sci USA, 94: 2150-2155 (1997)).

A DNA microarray is comprised of an orderly arrangement of polynucleotide or oligonucleotide samples. This technology allows a medium for matching known and unknown DNA samples based on base-pairing rules and automating the process of identifying unknowns. A DNA microarray can be fabricated by high-speed robotics or created manually, generally on glass but sometimes on nylon or other substrates, for which cDNA probes with known identity are used to determine complementary binding, and therefore allowing massive parallel gene expression and gene discovery studies. An experiment with a single DNA chip can provide information on thousands of genes simultaneously. (Schena, M. et al., Science, 270: 467-470 (1995); Shalon, D. et al., Genome Res., 6(7): 639-645 (1996)).

Using DNA microarrays, test and control mRNA samples from test and control samples are reverse transcribed and labeled to generate cDNA probes. The probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If such hybridization of a probe from a test (disease tissue) sample is greater than hybridization of a probe from a control (non-diseased tissue) sample, the gene or genes expressed in the diseased tissue are identified. Alternatively, the microarray can be used to identify genes which are expressed at higher levels on one cell type as compared to another cell type.

Detection sensitivity is a limiting factor for effectively analyzing test versus control samples such that gene expression associated with the disease is recognized. For the study of human genes using DNA microarrays, successful analysis of many disease states requires sufficiently sensitive detection to work with limiting quantities of sample.

Thus, the DNA microarray can be designed to detect differential expression of known genes derived from a first cell type, with respect to expression of the same gene in a second cell type (typically the first cell type corresponds to non-diseased tissue and the second cell type corresponds to diseased tissue including tumor). Of particular importance is the instance where altered expression of a specific gene is detected in diseased tissue compared to normal tissue, wherein such genes can then be used as targets for drug intervention for the reported disease. DNA microarray technology is thus very suitable for profiling diseases and for identifying disease related genes leading to the development of therapeutics and disease therapies for improved treatment of complex chronic diseases.

Differential and/or altered gene expression can be measured using a DNA microarray to monitor the expression level of large numbers of genes of interest which encode polypeptide antigens. The DNA microarray is used to monitor the expression level of large numbers of genes simultaneously (to produce a transcript image) and to identify genetic variants, mutations and polymorphisms. Specifically, the DNA microarray can be designed to detect differential expression of genes encoding polypeptide antigens derived from a first cell type, with respect to expression of the same gene in a second cell type (typically the first cell type corresponds to normal tissue and the second cell type corresponds to diseased tissue). Fluorescent-labeled cDNAs from mRNAs are isolated from the two cell types, where the cDNAs from the first and second cell types are labeled with first and second different fluorescent reporters. In a preferred embodiment, the microarray can be used to identify genes which are expressed at higher levels on one cell type as compared to another cell type.

In one embodiment, the DNA microarray is prepared and used according to the methods known in the art, such as those described in WO95/11995 (Chee et al., Lockart, D. J., et al., Nat. Biotech., 14:1675-1680 (1996), and Schena, M. et al., Proc. Natl. Acad. Sci., 93: 10614-10619 (1996)).

The DNA microarray is preferably composed of a large number of unique, single stranded nucleic acid sequences, usually either synthetic antisense oligonucleotides or fragments ofcDNAs, fixed to a solid support. The oligonucleotides are preferably about 6-60 nucleotides in length, more preferably about 15 to 30 nucleotides in length, and most preferably about 20 to 25 nucleotides in length. For a certain type of DNA microaray, it may be preferable to use oligonucleotides that are only 7 to 10 nucleotides in length. The DNA microarray may contain oligonucleotides which cover the known 5' (or 3') sequence, or may contain sequential oligonucleotides which cover the full-length sequence; or unique oligonucleotides selected from particular areas along the length of the sequence. Polynucleotides used in the DNA microarray may be oligonucleotides that are specific to a gene or genes of interest in which at least a fragment of the sequence is known or that are specific to one or more unidentified cDNAs that are common to a particular cell or tissue type or to a normal, developmental, or diseased state. In certain situations, it may appropriate to use pairs of oligonucleotides on a microarray. The pairs will be identical, except for one nucleotide preferably located in the center of the sequence. The second oligonucleotide in the pair (mismatched by one) serves as a control. The number of oligonucleotide pairs may range from 2 to 1,000,000.

For producing oligonucleotides to a known sequence for a microarray, the gene encoding a polypeptide antigen of interest is examined using a computer algorithm which starts at the 5' or more preferably at the 3' end of the nucleotide sequence. The algorithm identifies oligomers of defined length that are unique to the gene, have a GC content within a range suitable for hybridization, and lack predicted secondary structure that may interfere with hybridization.

In one aspect, the oligonucleotides may be synthesized at designated areas on the surface of a substrate by using a light-directed chemical coupling procedure and an inkjet application apparatus, such as that described in WO95/251116 (Balderschweiler et al.). The substrate may be paper, nylon or any other type of membrane, filter, chip, glass slide, or any other suitable solid support. In another aspect, a "gridded" array analogous to a dot or slot blot (HYBRIDOT^{®} apparatus, GIBCO/BRL) may be used to arrange and link cDNA fragments or oligonucleotides to the surface of a substrate using a vacuum system, thermal, UV, mechanical or chemical bonding procedures. In yet another aspect, an array may be produced by hand or by using available devices, materials, and machines (including BRINICMAN^{®} multichannel pipettors or robotic instruments). Such an array may contain 8, 24, 96, 384, 1536, or 6144 oligonucleotides, or any other multiple from 2 to 1,000,000 that lends itself to the efficient use of commercially available instrumentation.

In another aspect, the invention involves improved methods for generating fluorescently labeled cDNA probes from small quantities of nucleic acids, particularly ribonucleic acids. In mammalian tissue, for example, approximately 1% of the total RNA is messenger RNA/polyA+ RNA. Because mRNA/polyA+ RNA is the material providing the initial template for cDNA probe synthesis, it is available in very small amounts against a complex background of non-messenger RNAs (ribosomal RNA, transfer RNA, and the like). Consequently, the method of the invention for cDNA probe synthesis provides an advantage because the quantities of RNA useful as a template according to the present invention are 100-1000 fold less than the amounts useful in previously known methods.

In one embodiment, the method for generating fluorescently labeled cDNA probes involves the use of nanogram quantities of total cellular RNA. Such small amounts of total RNA are equivalent to picogram quantities of cellular messenger RNA, where mRNA is the actual template for reverse transcription to cDNA. Additional embodiments of the invention include generating fluorescently labeled cDNA probes from RNA isolated from diseased human tissues, microdissected tumor cells from diseased tissues, and formalin-fixed paraffin-embedded diseased tissue samples.

Sample analysis using the DNA microarray may be conducted by extracting polynucleotides from a biological sample. The biological samples may be obtained from any bodily fluid (blood, urine, saliva, phlegm, gastric juices, etc.), cultured cells, biopsies, or other tissue preparations. The polynucleotides from the sample may be used to produce, as probes, nucleic acid sequences that are complementary to the nucleic acids on the microarray. If the microarray consists of cDNAs, antisense RNAs (aRNA) are appropriate probes. Therefore, in one aspect, mRNA is used to produce cDNA that, in turn and in the presence of fluorescent nucleotides, is used to produce fragment or oligonucleotide aRNA probes. These fluorescently-labeled probes are incubated with the DNA microarray so that the probe sequences hybridize to the cDNA oligonucleotides of the microarray. In another aspect, nucleic acid sequences used as probes can include cDNA oligonucleotides, fragments, and complementary or antisense sequences produced using restriction enzymes, PCR technologies, and OLIGOLABELING^{™} or TRANSPROBE^{™} kits (Pharmacia) well known in the area of hybridization technology.

Incubation conditions are adjusted so that hybridization occurs with precise complementary matches or with various degrees of less complementarity. After removal of nonhybridized probes, a scanner is used to determine the levels and patterns of fluorescence. The scanned images are examined to determine degree of complementarity and the relative abundance of each oligonucleotide sequence on the microarray. A detection system may be used to measure the absence, presence, and amount of hybridization for all the distinct sequences (in this instance antigen encoding genes) simultaneously. This data may be used for large-scale correlation studies or functional analysis of the sequences, mutations, variants, or polymorphisms among samples. (Heller, R. A., et al., Proc. Natl. Acad. Sci. 94: 2150-55 (1997)). In addition, the data can be used to identify polypeptide antigens which are expressed more highly on the surface of proliferating cancer cells than on proliferating non-cancer cells.

### 5.2.11.2. Additional Techniques for Analysis of Expression Levels

Gene amplification and/or expression may be measured in a sample directly, for example, by conventional Southern blotting, Northern blotting to quantitate the transcription of mRNA [Thomas, Proc. Natl. Acad. Sci. USA. 77:5201-5205 (1980)], dot blotting (DNA analysis), or *in situ* hybridization, using an appropriately labeled probe. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal, as was discussed more fully above.

### 5.2.11.2.1. Purification of Polypeptide

Forms of polypeptides may be recovered from culture medium or from host cell lysates. If membrane-bound, they can be released from the membrane using a suitable detergent solution (*e.g*., Triton-X 100) or by enzymatic cleavage. Cells employed in expression of the polypeptide can be disrupted by various physical or chemical means, such as freeze-thaw cycling, sonication, mechanical disruption or cell lysing agents.

It may be desired to purify the polypeptide from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the polypeptide. Various methods ofprotein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular polypeptide produced.

### 5.2.11.2.2. Amplification of Genes Encoding Polypeptide Antigens

The present invention is based on the identification of genes that are amplified in certain cancer cells and thus, can be used as targets for cancer therapeutics. In a preferred embodiment, identified genes encoding polypeptide antigens are expressed more highly on the surface of proliferating cancer cells than on proliferating non-cancer cells.

The genome of prokaryotic and eukaryotic organisms is subjected to two seemingly conflicting requirements. One is the preservation and propagation of DNA as the genetic information in its original form, to guarantee stable inheritance through multiple generations. On the other hand, cells or organisms must be able to adapt to lasting environmental changes. The adaptive mechanisms can include qualitative or quantitative modifications of the genetic material. Qualitative modifications include DNA mutations, in which coding sequences are altered resulting in a structurally and/or functionally different protein. Gene amplification is a quantitative modification, whereby the actual number of complete coding sequence, *i.e*., a gene, increases, leading to an increased number of available templates for transcription, an increased number of translatable transcripts, and, ultimately, to an increased abundance of the protein encoded by the amplified gene.

The phenomenon of gene amplification and its underlying mechanisms have been investigated *in vitro* in several prokaryotic and eukaryotic culture systems. The best-characterized example of gene amplification involves the culture of eukaryotic cells in medium containing variable concentrations of the cytotoxic drug methotrexate (MTX). MTX is a folic acid analogue and interferes with DNA synthesis by blocking the enzyme dihydrofolate reductase (DHFR). During the initial exposure to low concentrations of MTX most cells (>99.9%) will die. A small number of cells survive, and are capable of growing in increasing concentrations of MTX by producing large amounts of DHFR-RNA and protein. The basis of this overproduction is the amplification of the single DHFR gene. The additional copies of the gene are found as extrachromosomal copies in the form of small, supernumerary chromosomes (double minutes) or as integrated chromosomal copies.

Gene amplification is most commonly encountered in the development of resistance to cytotoxic drugs (antibiotics for bacteria and chemotherapeutic agents for eukaryotic cells) and neoplastic transformation. Transformation of a eukaryotic cell as a spontaneous event or due to a viral or chemical/environmental insult is typically associated with changes in the genetic material of that cell. One of the most common genetic changes observed in human malignancies are mutations of the p53 protein. p53 controls the transition of cells from the stationary (G1) to the replicative (S) phase and prevents this transition in the presence of DNA damage. In other words, one of the main consequences of disabling p53 mutations is the accumulation and propagation of DNA damage, *i.e*., genetic changes. Common types of genetic changes in neoplastic cells are, in addition to point mutations, amplifications and gross, structural alterations, such as translocations.

The amplification of DNA sequences may indicate a specific functional requirement as illustrated in the DHFR experimental system. Therefore, the amplification of certain oncogenes in malignancies points toward a causative role of these genes in the process of malignant transformation and maintenance of the transformed phenotype. This hypothesis has gained support in recent studies. For example, the *bcl-2* protein was found to be amplified in certain types of non-Hodgkin's lymphoma. This protein inhibits apoptosis and leads to the progressive accumulation of neoplastic cells. Members of the gene family of growth factor receptors have been found to be amplified in various types of cancers suggesting that overexpression of these receptors may make neoplastic cells less susceptible to limiting amounts of available growth factor. Examples include the amplification of the androgen receptor in recurrent prostate cancer during androgen deprivation therapy and the amplification of the growth factor receptor homologue ERB2 in breast cancer. Lastly, genes involved in intracellular signaling and control of cell cycle progression can undergo amplification during malignant transformation. This is illustrated by the amplification of the *bcl-I* and *ras* genes in various epithelial and lymphoid neoplasms.

These earlier studies illustrate the feasibility of identifying amplified DNA sequences in neoplasms, because this approach can identify genes important for malignant transformation. The case of ERB2 also demonstrates the feasibility from a therapeutic standpoint, since transforming proteins may represent novel and specific targets for tumor therapy.

Several different techniques can be used to demonstrate amplified genomic sequences. Classical cytogenetic analysis of chromosome spreads prepared from cancer cells is adequate to identify gross structural alterations, such as translocations, deletions and inversions. Amplified genomic regions can only be visualized, if they involve large regions with high copy numbers or are present as extrachromosomal material. While cytogenetics was the first technique to demonstrate the consistent association of specific chromosomal changes with particular neoplasms, it is inadequate for the identification and isolation of manageable DNA sequences. The more recently developed technique of comparative genomic hybridization (CGH) has illustrated the widespread phenomenon of genomic amplification in neoplasms. Tumor and normal DNA are hybridized simultaneously onto metaphases of normal cells and the entire genome can be screened by image analysis for DNA sequences that are present in the tumor at an increased frequency. (WO 93/18,186; Gray et al., Radiation Res., 137:275-289[1994]). As a screening method, this type of analysis has revealed a large number of recurring amplicons (a stretch of amplified DNA) in a variety of human neoplasms. Although CGH is more sensitive than classical cytogenetic analysis in identifying amplified stretches of DNA, it does not allow a rapid identification and isolation of coding sequences within the amplicon by standard molecular genetic techniques. However, CGH can effectively be used to identify polypeptide antigens which are more highly expressed on the surface of proliferating cancer cells than on proliferating non-cancer cells.

The most sensitive methods to detect gene amplification are polymerase chain reaction (PCR)-based assays. These assays utilize very small amount of tumor DNA as starting material, are exquisitely sensitive, provide DNA that is amenable to further analysis, such as sequencing and are suitable for high-volume throughput analysis.

The above-mentioned assays are not mutually exclusive, but are frequently used in combination to identify amplifications in neoplasms. While cytogenetic analysis and CGH represent screening methods to survey the entire genome for amplified regions, PCR-based assays are most suitable for the final identification of coding sequences, *i.e*., genes in amplified regions.

According to the present invention, such genes encoding polypeptide antigens can be identified by quantitative PCR (S. Gelmini et al., Clin. Chem., 43:752 [1997]), by comparing DNA from a variety of primary tumors, including breast, lung, colon, prostate, brain, liver, kidney, pancreas, spleen, thymus, testis, ovary, uterus, etc., tumor, or tumor cell lines, with pooled DNA from healthy donors. Quantitative PCR can be performed using a TaqMan^{™} instrument (ABI). Gene-specific primers and fluorogenic probes will be designed based upon the coding sequences of the DNAs.

### 5.2.11.2.3. Tissue Distribution

The results of the gene amplification assays herein can be verified by further studies, such as, by determining mRNA expression in various human tissues.

As noted before, gene amplification and/or gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. General techniques for generating antibodies, and special protocols for Northern blotting and *in situ* hybridization have been provided herein and/or are well known in the art.

Thus, numerous techniques, as discussed above, currently exist which can be used for the identification of polypeptide antigens that are more highly expressed on one cell type versus another cell type. In a preferred aspect, these techniques can be used to identify polypeptide antigens which are more highly expressed on the surface of proliferating cancer cells than on the surface of proliferating non-cancer cells and which, therefore, can be used as targets for the identification, characterization and production of cancer therapeutics.

The following Examples are offered for illustrative purposes only.

### 6. EXAMPLES

Commercially available reagents referred to in the Examples were used according to manufacturer's instructions unless otherwise indicated. The source of those cells identified in the following Examples, and throughout the specification, byATCC accession numbers is the AmericanTypeCulture Collection, Manassas, VA. Unless otherwise noted, the present invention uses standard procedures of recombinant DNA technology, such as those described hereinabove and in the following textbooks: Sambrook *et al., supra;* Ausubel et al., Current Protocols in Molecular Biology (Green Publishing Associates and Wiley Interscience, N.Y.,1989); Innis et al., PCR Protocols: A Guide to Methods and Applications (Academic Press, Inc.: N.Y.,1990); Harlow et al., Antibodies: A Laboratory Manual (Cold Spring Harbor Press: Cold Spring Harbor,1988); Gait, Oligonucleotide Synthesis (IRL. Press: Oxford,1984); Freshney, Animal Cell Culture 1987; Coligan et al., Current Protocols in Immunology, 1991.

### 6.1. Example 1: HERCEPTIN^{®}-DM1ConiuR8tes

### 6.1.1. Purification of HERCEPTTN^{®}

HERCEPTIN^{®} (huMAb4D5-8, rhuMAb HER2, U.S. Patent No. 5,821,337) (1 vial containing 440 mg antibody) was dissolved in 50 mL MES buffer (25 mM MES, 50 mM NaCl, pH 5.6). The sample was loaded on a cation exchange column (Sepharose S,15 cm x 1.7 cm) that had been equilibrated in the same buffer. The column was then washed with the same buffer (5 column volumes). HERCEPTIN^{®} was eluted by raising the NaCl concentration of the buffer to 200 mM. Fractions containing the antibody were pooled, diluted to 10 mg/mL, and dialyzed into a buffer containing 50 mm potassium phosphate, 50 mM NaCl, 2 mM EDTA, pH 6.5.

### 6.1.2. Modification of HERCEPTIN^{®} with SPP

The purified HERCEPTIN^{®} antibody was modified with N-succinimidyl-4-(2-pyndylthio)pentanoate (SPP) to introduce dithiopyridyl groups. The antibody (376.0 mg, 8 mg/mL) in 44.7 mL of 50 mM potassium phosphate buffer (pH 6.5) containing NaCl (50 mM) and EDTA (1 mM) was treated with SPP (5.3 molar equivalents in 2.3 mL ethanol). After incubation for 90 minutes under argon at ambient temperature, the reaction mixture was gel filtered through a Sephadex G25 column equilibrated with 35 mM sodium citrate, 154 mM NaCl, 2 mM EDTA. Antibody containing fractions were pooled and assayed. The degree of modification of the antibody was determined as described above. Recovery of the modified antibody (HERCEPTIN^{®}-SPP-Py) was 337 mg (89.7%) with 4.5 releasable 2-thiopyridine groups linked per antibody.

### 6.1.3. Conjugation of HERCEPTIN^{®}-SPP-Py with DM1

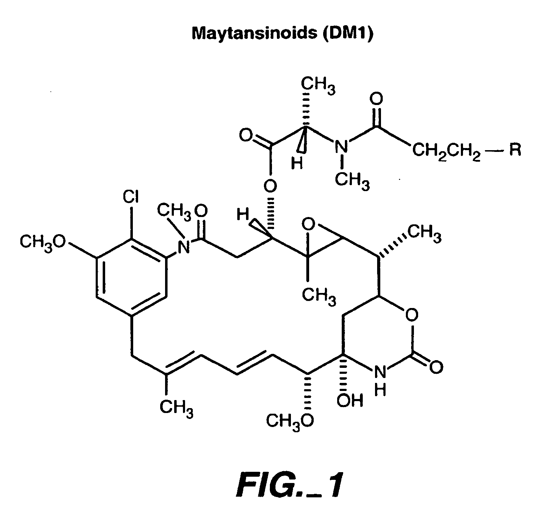

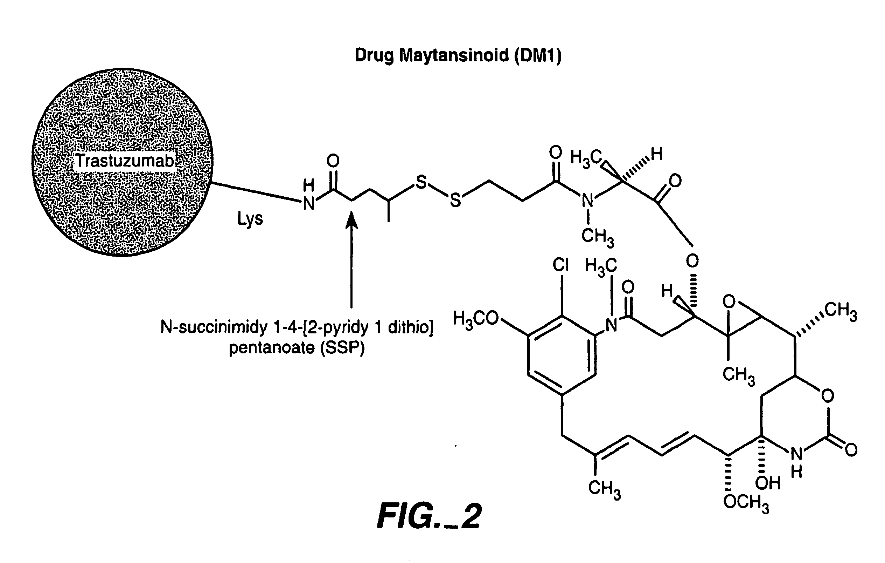

The modified antibody (337.0 mg, 9.5 µmols of releasable 2-thiopyridine groups) was diluted with the above 35 mM sodium citrate buffer, pH 6.5, to a final concentration of 2.5 mg/mL. DM1 (1.7 equivalents, 16.1 µmols) in 3.0 mM dimethylacetamide (DMA, 3% v/v in the final reaction mixture) was then added to the antibody solution. The structure of DM1 is shown in Figure 1, where the nature of the "R" group is not critical and can be occupied, for example, by a variety of groups capable of forming a chemical bond with a linker. DM 1 used in the present reaction was stored as an S-S form, which is more stable, and was reduced to the SH form for conjugation with the HERCEPTIN^{®} antibody. The reaction proceeded at ambient temperature under argon for 20 hours. The structure of HERCEPTITI^{®}-DM 1 conjugates is illustrated in Figure 2.

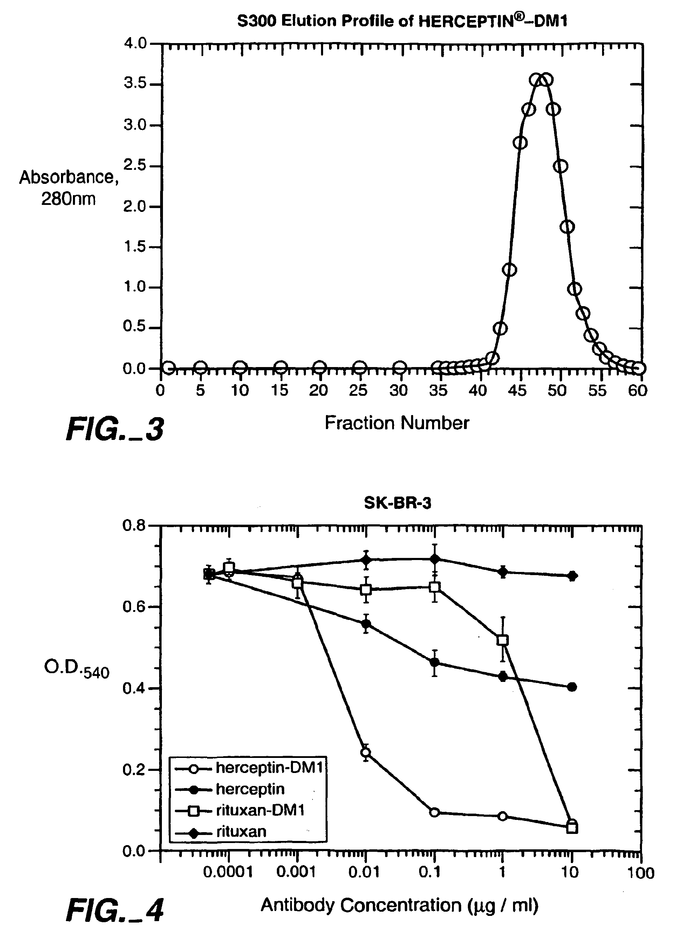

The reaction was loaded on a Sephacryl S300 gcl filtration column (5.0 cm x 90.0 cm, 1.77 L) equilibrated with 35 mM sodium citrate, 154 mM NaCl, pH 6.5. The flow rate was 5.0 mL/min and 65 fractions (20.0 mL each) were collected. A major peak centered around fraction No. 47 (Figure 3). The major peak comprises monomeric HERCEPTIN^{®}-DM1. Fractions 44-51 were pooled and assayed. The number of DM1 drug molecules linked per antibody molecule was determined by measuring the absorbance at 252 nm and 280 nm, and found to be 3.7 drug molecules per antibody molecule.

### 6.1.4. Anti-proliferative Effect of HERCEPTIN^{®}-DM1 Conjugate in vitro

SK-BR3 cells, which express 3+ level of HER2 on cell surface (about 2 million HER2 molecules /cell), were treated with HERCEPITN^{®}, HERCEPTIN^{®}-DM1 conjugate, control mAb RITUXAN^{®} or RITUXAN^{®}-DM1 conjugates, and the effect of these treatments on cell proliferation was monitored. As shown in Fig. 4, the extent of cell growth inhibition by treatment with HERCEPTIN^{®}-DM 1 was dramatically more pronounced than that with HERCEPTIN^{®}, while the control RITUXAN^{®} antibody did not inhibit cell growth. Although the RITUXAN^{®}-DM 1 did inhibit cell growth, it did so only at high concentrations. For example, the RITUXAN^{®}-DMI conjugate did not significantly inhibit growth at concentrations up to 1 µg/ml. In contrast, the HERCEPTIN^{®}-DM1I conjugate was highly potent and significantly inhibited cell growth starting from 0.01 µg/ml and reaching a plateau at 0.1 µg/ml. The RITUXAN^{®}-DM1 I conjugate required about 100 times higher concentration to achieve the same level of cell growth inhibition as HERCEPTIN^{®}-DM1 conjugate. This is also reflected in a 100-fold difference in IC₅₀ value, concentration required to inhibit cell growth by 50%, of the respective conjugates.

### 6.2. Example 2: Lack of Toxicity with HERCEPTIN^{®}-DM1 Conjugates

The following experiment demonstrates the lack of in *vivo* toxicity associated with HERCEPTIN^{®}-DM 1 conjugates.

### 6.2.1. Experimental Design

HERCEPTIN^{®}-DM1 was administered to young adult female cynomolgus monkeys (*Macaca fascicularis*; Primate Products, Inc., Miami Florida) once weekly for four weeks. The average weight of the monkeys was three kilograms (range from 2.7 to 3.4 kilograms). A total of eight monkeys, divided into four groups of two monkeys each, were utilized for the study. The dosages of HERCEPTIN^{®}-DM1I tested were 2, 10 and 30 mg/kg. A control group received vehicle only (an aqueous buffer (pH 5.0) containing sodium succinate (10mM), sucrose (100 mg/ml) and Tween 20 (0.1%)) at the same dose volume as administered to the treated animals. Th monkeys were analyzed for various toxicities, including, but not limited to, neurotoxicity and cardiotoxicity. Table 2, below, more particularly gives the details of the experimental design for the instant experiment.

**TABLE 2**

| | | | | | | | | | |
|---|---|---|---|---|---|---|---|---|---|
| The test/control article was administered once weekly for 4 weeks via intravenous injection (slow bolus over 30-60 seconds). Animals were surgically implanted with telemetry probes for the collection of cardiovascular data using the ART (version 1.0) software package from DSI (Data Sciences International). Additional cardiovascular data was collected using 2D Doppler echocardiography. | | | | | | | | | |

| Group | Dose = 1x/week | | | Number of Animals (Female) | | | | | |
|---|---|---|---|---|---|---|---|---|---|
| | Dose (mg/kg) | Volume (mL/kg) | Conc. (mg/mL) | Total | Toxicokinetics¹ | Clinical Pathology² | Antibody Analysis³ | Necropsy (Week 4) | Microscopic Pathology |
| 1 | 0 | 6 | 0 | 2 | 2 | 2 | 2 | 2 | 2 |
| 2 | 2 | 6 | 0.333 | 2 | 2 | 2 | 2 | 1 | 2 |
| 3 | 10 | 6 | 1.67 | 2 | 2 | 2 | 2 | 2 | 2 |
| 4 | 30 | 6 | 5.0 | 2 | 2 | 2 | 2 | 2 | 2 |

| | | | | | | | | | |
|---|---|---|---|---|---|---|---|---|---|
| ¹Toxicokinetic samples were collected on Day 0:pre-dose, and post-dose at 1 hour, 48 hours, and 72 hours; Day 7:pre-dose, and post-dose at 1 hour; Day 14:pre-dose, and post-dose at 1 hour; Day 21:pre-dose, and post-dose at I hour, 8 hours, 24 hours, and 48 hours; Day 28 prior to terminal sacrifice. ²Hematology, clinical chemistry, including cardiac troponins (Troponin I and Troponin T) and creatine kinase isoenzymes were evaluated for all animals twice pretest and 5 days following each dose (Days 5, 12, 19, and 26). Cardiac troponins and creatine kinase isoenzymes were also evaluated approximately 6 hours after each dose administration (Days 0, 7, 14, and 21). ³Blood samples were collected for antibody analysis pretest, predose on Day 14, and at study termination (Day 28). mg/kg = milligrams of test article per kilogram of body weight. The first day of dosing is designated Day 0. | | | | | | | | | |

### 6.2.2. Animal Husbandry

### 6.2.2.1. Housing

Animals were pair-housed in elevated stainless steel cages during the quarantine period according to specifications of USDA Animal Welfare Act (8 CFR Parts 1, 2 and 3). Animals were individually housed from the time of surgery through study termination because of telemetry evaluations.

### 6.2.2.2. Feed

Certified Primate Diet, No. 5048 (PMI Nutrition International, St. Louis, Missouri). Fresh feed was presented once daily. The amount of feed presented was approximately 4% by weight of the mean body weight of the animals in the room. Diets were supplemented with fruits and vegetables three times per week. Analysis of each feed lot used during this study was performed by the manufacturer.

### 6.2.2.3. Water

Water was available without restriction via an automated watering system (Elizabethtown Water Company, Westfield, New Jersey). Water analyses were conducted by Elizabethtown Water Company, Westfield, New Jersey (Raritan-East Millstone Plant) to ensure that water met standards specified under the EPA Federal Safe Drinking Water Act Regulations (40 CFR Part 141). In addition, water samples were collected biannually from representative rooms in the Testing Facility; chemical and microbiological water analyses were conducted on these samples by a subcontract laboratory.

### 6.2.2.4. Environmental Conditions

Twelve hour light/dark cycle controlled via an automatic timer. Light cycles were interrupted as necessary for collection of toxicokinetic blood samples.

Temperature was monitored and recorded twice daily and maintained within the specified range to the maximum extent possible. The range of temperature was 17°C to 29°C.

Relative humidity was monitored and recorded once daily and maintained within the specified range to the maximum extent possible. The range of humidity was 20% to 80%.

### 6.2.3. HERCEPTIN^{®}-DM1 Administration

### 6.2.3.1. Route of Administration

The test and control articles were administered over a 30 to 60 second time period by intravenous injection into an indwelling catheter inserted into the saphenous or cephalic vein, using a stainless steel dosing needle and syringe of appropriate size. The indwelling catheter was flushed with sterile saline prior to use and tested to ensure that it was inserted properly in the vein. After dosing, the catheter was flushed with approximately 2 ml of sterile saline. Doses were calculated using the most recent body weights available.

### 6.2.3.1. Frequency, Duration and Level of Dosing

The test article was administered once weekly for four weeks (on Days 0, 7, 14 and 21). Dosage levels were as follows: Group 1 - 0 mg/kg; Group 2 - 2 mg/kg; Group 3 - 10 mg/kg and Group 4 - 30 mg/kg. All dosages were administered at a volume of 6 ml/kg.

### 6.2.4. Surgical Preparation of Animals

### 6.2.4.1. Telemetry Probe Implantation

Monkeys were treated prophylactically with antibiotic (Baytril® a DNA gyrase inhibitor, 5 mg/kg, intramuscularly) on the day prior to surgery, on the day of surgery and for 5 days following surgery. Animals were fasted for a minimum of 12 hours prior to surgery, but not more than 18 hours total (including duration of surgery). Animals were treated prior to anesthesia with ketamine (10 mg/kg) and atropine (0.05 mg/kg). Anesthesia was induced and maintained by isoflurane. The animals were maintained under the anesthesia for the entire surgical period. Once anesthetized, the appropriate abdominal and inguinal regions were shaved and prepared for the surgical implantation procedures. A small incision was made in the left inguinal region and peritoneal cavity. The telemetry device/transmitter (Data Sciences International, St. Paul, MN) was positioned within the peritoneal cavity and secured via suture to either the omentum or the peritoneal mucosal surface (an animal dependent selection). The blood pressure catheter of the telemetry probe was exteriorized through the abdominal musculature and run subcutaneously to the left groin region. The blood pressure catheter was inserted into the left femoral artery, with the catheter tip placed into the abdominal aorta and secured with sutures. The electrocardiogram leads were also exteriorized and subcutaneously tunneled to the appropriate anatomical region. Both leads were then secured with sutures.

### 6.2.4.1. Postsurgical Procedures

An analgesic, flunixin meglumine, a non-steroidal anti-inflammatory agent (1 mg/kg, intramuscularly), was administered immediately after surgery (prior to recovery from anesthesia). Additional flunixin meglumine was administered as deemed necessary by the Study Director and/or the staff veterinarian. During the post-operative period on the day of the surgical procedure, animals were observed until they had recovered from anesthesia, and feed was presented overnight. Monkeys were treated prophylactically with an antibiotic, (Baytril®, 5 mg/kg, intramuscularly) for 5 days following surgery. Monkeys were allowed at least 14 days to recover prior to the initiation of dosing. No handling of the animals was performed for at least 7-10 days post surgery in order to avoid potential opening of the abdominal sutures.

### 6.2.5. Summary and Conclusions

The present experiment was designed to assess the potential toxic (*e.g*., cardiotoxicity and neurotoxicity) effects of HERCEPTIN^{®}-DM1 at doses of 2, 10 or 30 mg/kg administered to female cynomolgus monkeys via intravenous injection once weekly for four weeks. A control group (2 animals) received the vehicle (an aqueous buffer (pH 5.0) containing sodium succinate (10 mM), sucrose (100 mg/ml) and Tween 20 (0.1 %)) at the same dose volume as administered to the treated animals.

Physical observations were performed twice pretest and once weekly during the study period. Body weights were recorded twice pretest, weekly during the study period and prior to termination. Blood samples for toxicokinetic analysis were collected at selected intervals on the days of test article administration and at study termination. Blood samples for antibody analysis were collected pretest, predose on Day 14, and at study termination. Hematology and clinical chemistry, including assays for creatine kinase isoenzymes and cardiac troponins (troponin I and troponin T), were performed twice pretest and on Days 5, 12, 19 and 26. Samples for creatine kinase isoenzymes and cardiac troponins were also collected approximately 6 hours post-dose on Days 0, 7, 14 and 21.

Cardiovascular assessments were performed radiotelemetrically for two 24-hour periods pretest. On days of test article administration, animals were monitored for approximately two hours predose, every minute for 4 hours post-dose, every 30 minutes from 4 hours post-dose to 24 hours post-dose and every hour for the remainder of the dose week. Manual 9-lead electrocardiograms were recorded twice pretest and at termination of the dosing period. Echocardiogram measurements were conducted three times pretest and on Days 5, 12, 19 and 26.

After four weeks of treatment, all survivors were sacrificed. Complete macroscopic postmortem examinations and histopathological evaluation of selected tissues were conducted on all animals. Cardiac and nerve tissues were collected from each animal and analyzed.

There was no HERCEPTIN^{®}-DM1- related mortality during the study. There were no HERCEPTIN^{®}-DMI-related clinical findings or effects on body weights. There was no electrocardiographic evidence of HERCEPTIN^{®} -DM1-related toxicity ineither multilead electrocardiograms recorded at termination or in single lead telemetered electrocardiograms recorded at frequent intervals throughout the study. There was no evidence of progressive deterioration of left ventricular function in animals at any dose (2, 10 or 30 mg/kg) when several 2D/M-mode cardiac dimensions and Doppler blood flow variables were measured by echocardiography five days following each dose. There was no indication of an effect of HERCEPTIN^{®}-DM1 on blood pressure values (mean, systolic, and diastolic) at doses of 2, 10, or 30 mg/kg. There was no clear evidence of a HERCEPTIN^{®}-DM1-related effect on hematology values. There were no HERCEPTIN^{®}-DM1-related effects on clinical chemistry values or on serum levels of creatine kinase MB and the cardiac troponins I and T, which are specific markers for cardiac damage. There were no macroscopic findings attributable to treatment with the HERCEPTIN^{®}-DM1. However, HERCEPTIN^{®}-DM1-related microscopic findings did include microscopic neurodegenerative changes in the sciatic and vagus nerves, as well as secondary effects in skeletal muscle, for animals treated at the high dose level of 30 mg/kg. The 30 mg/kg level, however, represents about a 10-fold increase in the expected therapeutic level. Furthermore, based on cage-side observations, no neurological or epithelial cell related (*e.g*. gastointestinal, skin, infection, *etc.*) changes occurred following treatment with HERCEPTIN^{®}-DM1.

In conclusion, based on clinical evaluations, measurement of specific serum markers for cardiac lesions and examination of heart tissue by light microscopy, there was no evidence of cardiotoxicity in female cynomolgus monkeys treated with Herceptin-DM1 at doses of 2, 10 or 30 mg/kg via intravenous injection once weekly for four weeks. Peripheral neuropathy was observed in female cynomolgus monkeys treated with HERCEPTIN^{®}-DM1 at doses of 30 mg/kg via intravenous injection once weekly for four weeks. However, as noted above, the 30 mg/kg level represents approximately a 10-fold increase over the expected therapeutic level. Moreover, no neurological or epithelial cell related (*e.g*. gastointestinal, skin, infection, *etc.*) changes appeared to occur following treatment with HERCEPTIN^{®}-DM1.

### 6.3. Example 3: HERCEPTIN^{®}-DM1 Conjugates are not Toxic to Normal Human Cells or to Growth-arrested Cells

The following experiment demonstrates the lack oftoxicity associated with HERCEPTIN^{®}-DM1 conjugates to normal human cells and to growth-arrested cells.

### 6.3.1. Experimental Design

Normal human mammary epithelial cells (HMEC), small airway epithelial cells (SAEC) and adult epidermal keratinocytes (NHEK) were obtained from Clonetics/BioWhittaker (San Diego, CA). Human hepatocytes were obtained from In Vitro Technologies (Baltimore, MD). SK-BR-3 human breast carcinoma cells were from The American Type Culture Collection (Rockville, MD). Culture media used were: MEGM (mammary epithelial cell growth media), SAGM (small airway epithelial cell growth media) and KGM (keratinocyte growth media), all from Clonetics/BioWhittaker; and hepatocyte incubation media (In Vitro Technologies). SK-BR-3 cells were cultured in high glucose DMEM:Ham's F-12 (50:50) supplemented with 10% heat-inactivated fetal bovine serum and 2 mM 1-glutamine (all from GIBCO/BRL, Grand Island, NY).

Cells were plated at the following densities in 96-well microtiter culture plates: 2x10⁴ per well for SK-BR-3; 10⁴ per well for HMEC, SAEC and NHEK; and 3.5x10⁴ per well for human hepatocytes (pre-plated on collagen-coated plates by In Vitro Technologies) and allowed to adhere overnight in the incubator (37°C, 5% CO₂). The following day, antibodies alone (Herceptin or Rituxan) or antibody-maytansinoid conjugates (HERCEPTIN^{®}-DM1 I or RITUXAN^{®}-DM1) were added at concentrations ranging from 0.1 ng/ml - 10 mg/ml. After a 3 day incubation, the media were removed, the cell monolayers washed once with PBS and stained with crystal violet dye (0.5% crystal violet, 20% methanol).

For experiments on growth-arrested cells, SK-BR-3 breast tumor cells were plated in 96-well microtiter plates in fully supplemented culture media at a density of either 5 x 10³ per well (for cells which will remain in media containing 10% FBS) or 2 x 10⁴ per well (for cells to undergo growth arrest). After an overnight incubation, media were removed and replaced with media supplemented with 10% FBS to allow for normal cell growth or media containing 0.1% FBS to initiate cell cycle arrest. Following a 3 day incubation to allow for complete growth arrest, media were again removed and replaced with media containing either 10% FBS or 0.1% FBS and Herceptin or antibody-DM 1 conjugates (0.1 ng/ml - 10 mg/ml). The cells were then incubated for 3 days and the monolayers stained with crystal violet dye as described above.

For all experiments, after removal of the crystal violet dye, plates were allowed to air-dry overnight. The dye was then eluted with 0.1 M sodium citrate:ethanol (50:50), pH 4.2 and the plates read in an SLT 340 ATC plate-reader at a wavelength of 540 nm. Each treatment group consisted of 4 replicates and the data are represented as mean O.D.₅₄₀ +/- standard error or relative to cell proliferation as compared to untreated control cells (mean +/- s.e.).

### 6.3.2. HERCEPTIN^{®}-DM1 Conjugate is not Toxic to Normal Cells

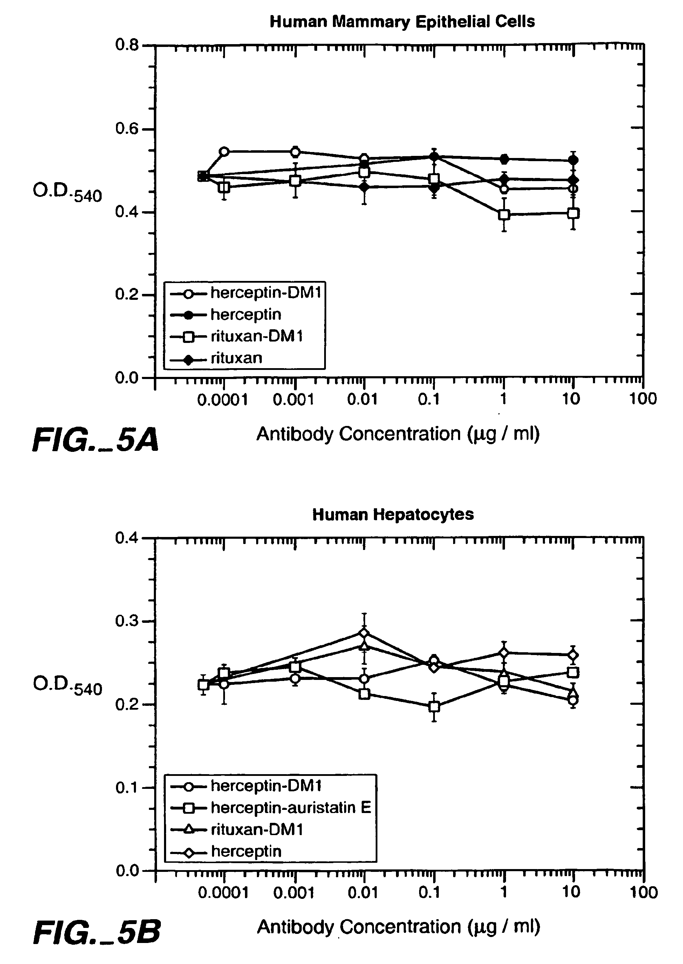

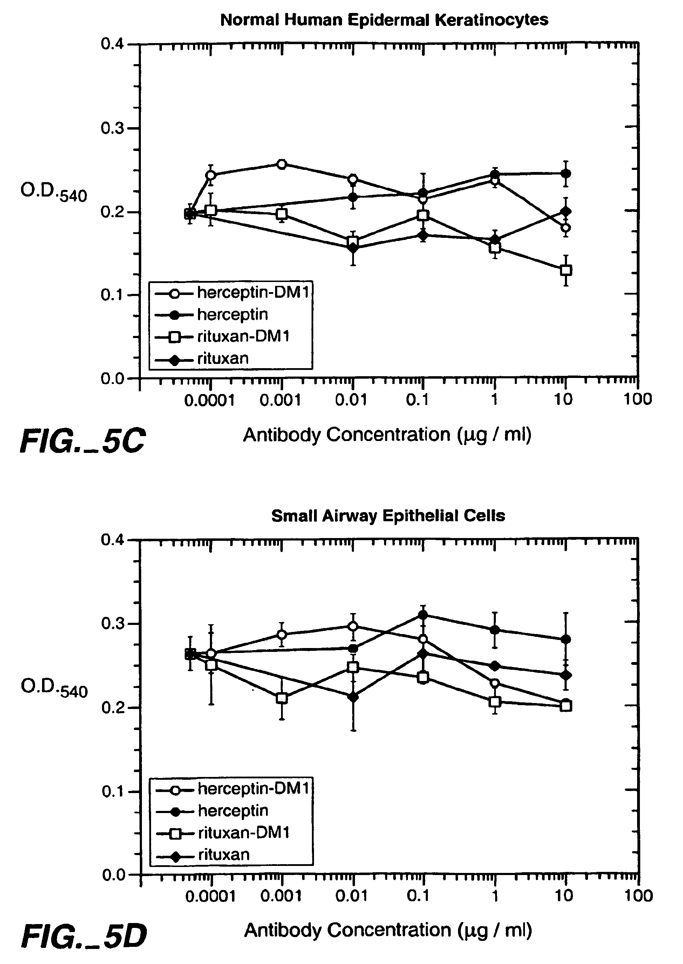

Treatment of SK-BR-3 breast tumor cells with HERCEPTIN^{®}-DM1 resulted in dose-dependent cytotoxicity, with an EC₅₀ of approximately 0.005 mg/ml (33 pM) (Fig. 4). Herceptin alone caused a modest reduction (35%) in SK-BR-3 cell growth. The control antibody-maytansinoid conjugate, RITLIXAN^{®}-DM1, showed toxicity only at the highest dose tested (10 µg/ml). In contrast, HERCEPTIN^{®}-DM1 had no effect on normal human mammary epithelial cells (Fig. 5A), normal human hepatocytes (Fig. 5B); normal human epidermal keratinocytes (Fig. 5C) or normal human small airway epithelial cells (Fig. 5D).

### 6.3.3. HERCEPTIN^{®}-DM1 Conjugate is not Toxic to Growth-aressted Cells

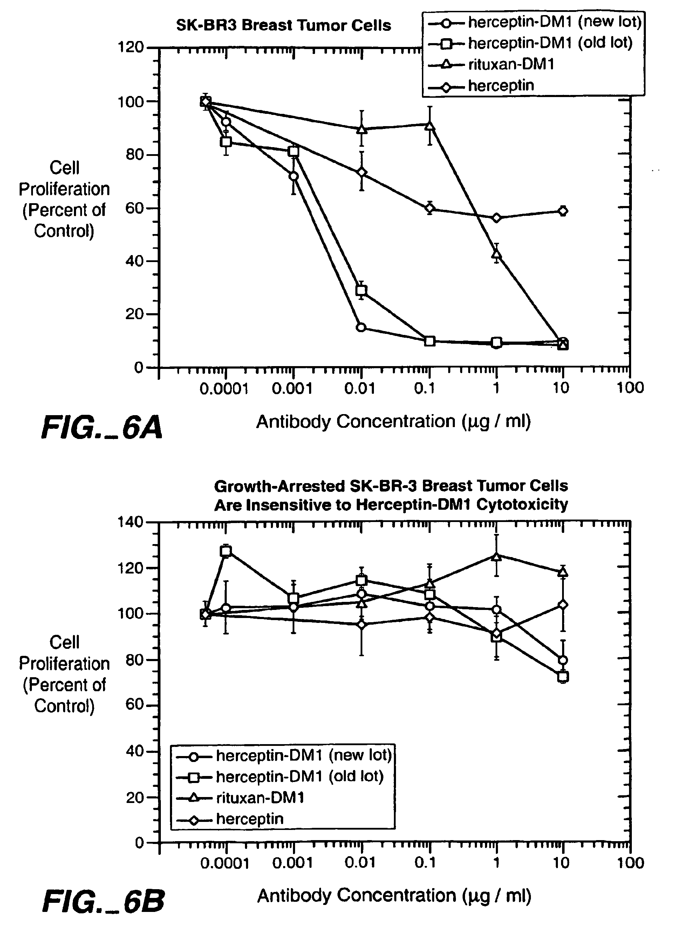

As noted above, treatment of SK-BR-3 breast tumor cells with HERCEPTIN^{®}-DM1 resulted in dose-dependent cytotoxicity (Fig. 4). Fig. 6A shows that treatment of SK-BR-3 breast tumor cells with HERCEPTIN^{®}-DM 1 resulted in dose-dependent decrease in cell proliferation. Herceptin alone caused a modest reduction in SK-BR-3 cell proliferation, whereas the control antibody-maytansinoid conjugate, RITUXAN^{®}-DM1, showed significant reduction in cell proliferation only at the highest dosages tested (1 and 10 µg/ml).

Following a period ofserum-deprivation which results in cellular growth arrest, treatment of SK-BR-3 cells with HERCEPTIN^{®}-DM1 did not result in a cytotoxic response. Growth-arrested SK-BR-3 breast tumor cells were completely resistant to HERCEPTIN^{®}-DM1 (or high dose RITUXAN^{®}-DM1) cytotoxicity, even at the highest dosages tested (10 µg/ml) (Fig. 6B). In addition, as mentioned above, human hepatocytes were insensitive to antibody-maytansinoid killing. As hepatocytes are non-dividing cells, these results further support the finding that HERCEPTIN^{®}-DM1 has no effect on non-dividing cells.

## Claims

1. A method for identifying a polypeptide antigen on the surface of a cell as a target for cancer therapy with an anti-mitotic compound, the method comprising identifying a polypeptide antigen as having a copy number at least 10% higher on the surface of a proliferating cancer cell than on the surface of a proliferating non-cancer cell of the same cell type;
wherein there is less than a 10% difference in copy number of the polypeptide antigen on the surface of a non-proliferating or slowly proliferating non-cancer cell of the same cell type as compared with the proliferating cancer cell,
or wherein the copy number of the polypeptide antigen on the surface of a non-proliferating or slowly proliferating non-cancer cell of the same cell type is at least 10% higher than on the surface of the proliferating cancer cell;
wherein the non-proliferating cell Is a cell in which M phase of the cell's reproductive cycle occurs less than every 72 hours, and wherein the slowly proliferating cell is a cell in which M phase of the cell's reproductive cycle occurs less than every 8 hours but more than or equal to every 72 hours;
thereby identifying said polypeptide antigen,

2. The method according to claim 1, wherein there is less than a 5% difference in copy number of the polypeptide antigen on the surface of the non-proliferating or slowly proliferating non-cancer cell as compared with the proliferating cancer cell.

3. The method according to claim 1, wherein the copy number of the polypeptide antigen on the surface of a non-proliferating or slowly proliferating non-cancer cell is at least 30% higher than on the surface of the proliferating cancer cell.

4. The method according to claim 3, wherein the copy number of the polypeptide antigen on the surface of a non-proliferating or slowly proliferating non-cancer cell is at least 60% higher than on the surface of the proliferating cancer cell.

5. The method according to claim 4, wherein the copy number of the polypeptide antigen on the surface of a non-proliferating or slowly proliferating non-cancer cell is at least 80% higher than on the surface of the proliferating cancer cell.

6. The method according to claims 5, wherein the copy number of the polypeptide antigen on the surface of a non-proliferating or slowly proliferating non-cancer cell Is at least 100% higher than on the surface of the proliferating cancer cell.

7. The method according to any of the preceding claims, wherein the polypeptide antigen has a copy number at least 30% higher on the surface of the proliferating cancer cell than on the surface of the proliferating non-cancer cell.

8. The method according to claim 7, wherein the polypeptide antigen has a copy number at least 60% higher on the surface of the proliferating cancer cell than on the surface of the proliferating non-cancer cell.

9. The method according to claim 8, wherein the polypeptide antigen has a copy number at least 80% higher on the surface of the proliferating cancer cell than on the surface of the proliferating non-cancer cell.

10. The method according to claim 9, wherein the polypeptide antigen has a copy number at least 100% higher on the surface of the proliferating cancer cell than on the surface of the proliferating non-cancer cell.

11. The method according to any of claims 1 to 6, wherein said polypeptide antigen has a copy number at least 10% higher on the surface of said proliferating cancer cell than on the surface of all proliferating non-cancer cell types,

12. The method according to claim 11, wherein said polypeptide antigen has a copy number at least 30% higher on the surface of said proliferating cancer cell than on the surface of all proliferating non-cancer cell types.

13. The method according to claims 12, wherein said polypeptide antigen has a copy number at least 60% higher on the surface of said proliferating cancer cell than on the surface of all proliferating non-cancer cell types.

14. The method according to claim 13, wherein said polypeptide antigen has a copy number at least 80% higher on the surface of said proliferating cancer cell than on the surface of all proliferating non-cancer cell types.

15. The method according to claim 14, wherein said polypeptide antigen has a copy number at least 100% higher on the surface of said proliferating cancer cell than on the surface of all proliferating non-cancer cell types.

16. The method according to any of the preceding claims, wherein said polypeptide antigen has a copy number at least 10% higher on the surface of a non-proliferating or slowly proliferating non-cancer cell than on the surface of a proliferating non-cancer cell.

17. The method according to claim 16, wherein said polypeptide antigen has a copy number at least 30% higher on the surface of a non-proliferating or slowly proliferating non-cancer cell than on the surface of a proliferating non-cancer cell.

18. The method according to claim 17, wherein said polypeptide antigen has a copy number at least 60% higher on the surface of a non-proliferating or slowly proliferating non-cancer cell than on the surface of a proliferating non-cancer cell.

19. The method according to claim 18, wherein said polypeptide antigen has a copy number at least 80% higher on the surface of a non-proliferating or slowly proliferating non-cancer cell than on the surface of a proliferating non-cancer cell.

20. The method according to claim 19, wherein said polypeptide antigen has a copy number at least 100% higher on the surface of a non-prolfferating or slowly proliferating non-cancer cell than on the surface of a proliferating non-cancer cell.

21. The method according to any of the preceding claims, wherein said step of identifying a polypeptide antigen comprises employing microarray analysis.

## Patentansprüche

1. Verfahren zur Identifizierung eines Polypeptidantigens an der Oberfläche einer Zelle als Ziel für die Krebstherapie mit einer antimitotischen Verbindung, wobei das Verfahren das Identifizieren eines Polypeptidantigens als eines umfasst, dessen Kopienanzahl an der Oberfläche einer proliferierenden Krebszelle um zumindest 10 % höher ist als an der Oberfläche einer proliferierenden Nicht-Krebs-Zelle desselben Zelltyps;
worin der Unterschied der Kopienanzahl des Polypeptidantigens an der Oberfläche einer nicht proliferierenden oder langsam proliferierenden Nicht-Krebs-Zelle desselben Zelltyps gegenüber der proliferierenden Krebszelle weniger als 10 % ausmacht
oder worin die Kopienanzahl des Polypeptidantigens an der Oberfläche einer nicht proliferierenden oder langsam proliferierenden Nicht-Krebs-Zelle desselben Zelltyps um zumindest 10 % höher ist als an der Oberfläche der proliferierenden Krebszelle;
worin die nicht proliferierende Zelle eine Zelle ist, bei der die M-Phase des Reproduktionszyklus der Zelle seltener als alle 72 h auftritt, und worin die langsam proliferierende Zelle eine Zelle ist, bei der die M-Phase des Reproduktionszyklus der Zelle seltener als alle 8 h, aber öfter als oder genau alle 72 h auftritt;
wodurch das Polypeptidantigen identifiziert wird.

2. Verfahren nach Anspruch 1, worin der Unterschied der Kopienanzahl des Polypeptidantigens an der Oberfläche der nicht proliferierenden oder langsam proliferierenden Nicht-Krebs-Zelle gegenüber der proliferierenden Krebszelle weniger als 5 % ausmacht.

3. Verfahren nach Anspruch 1, worin die Kopienanzahl des Polypeptidantigens an der Oberfläche einer nicht proliferierenden oder langsam proliferierenden Nicht-Krebs-Zelle um zumindest 30 % höher ist als an der Oberfläche der proliferierenden Krebszelle.

4. Verfahren nach Anspruch 3, worin die Kopienanzahl des Polypeptidantigens an der Oberfläche einer nicht proliferierenden oder langsam proliferierenden Nicht-Krebs-Zelle um zumindest 60 % höher ist als an der Oberfläche der proliferierenden Krebszelle.

5. Verfahren nach Anspruch 4, worin die Kopienanzahl des Polypeptidantigens an der Oberfläche einer nicht proliferierenden oder langsam proliferierenden Nicht-Krebs-Zelle um zumindest 80 % höher ist als an der Oberfläche der proliferierenden Krebszelle.

6. Verfahren nach Anspruch 5, worin die Kopienanzahl des Polypeptidantigens an der Oberfläche einer nicht proliferierenden oder langsam proliferierenden Nicht-Krebs-Zelle um zumindest 100 % höher ist als an der Oberfläche der proliferierenden Krebszelle.

7. Verfahren nach einem der vorangegangenen Ansprüche, worin die Kopienanzahl des Polypeptidantigens an der Oberfläche der proliferierenden Krebszelle um zumindest 30 % höher ist als an der Oberfläche der proliferierenden Nicht-Krebs-Zelle.

8. Verfahren nach Anspruch 7, worin die Kopienanzahl des Polypeptidantigens an der Oberfläche der proliferierenden Krebszelle um zumindest 60 % höher ist als an der Oberfläche der proliferierenden Nicht-Krebs-Zelle.

9. Verfahren nach Anspruch 8, worin die Kopienanzahl des Polypeptidantigens an der Oberfläche der proliferierenden Krebszelle um zumindest 80 % höher ist als an der Oberfläche der proliferierenden Nicht-Krebs-Zelle.

10. Verfahren nach Anspruch 9, worin die Kopienanzahl des Polypeptidantigens an der Oberfläche der proliferierenden Krebszelle um zumindest 100 % höher ist als an der Oberfläche der proliferierenden Nicht-Krebs-Zelle.

11. Verfahren nach einem der Ansprüche 1 bis 6, worin die Kopienanzahl des Polypeptidantigens an der Oberfläche der proliferierenden Krebszelle um zumindest 10 % höher ist als an der Oberfläche aller proliferierenden Nicht-Krebs-Zelltypen.

12. Verfahren nach Anspruch 11, worin die Kopienanzahl des Polypeptidantigens an der Oberfläche der proliferierenden Krebszelle um zumindest 30 % höher ist als an der Oberfläche aller proliferierenden Nicht-Krebs-Zelltypen.

13. Verfahren nach Anspruch 12, worin die Kopienanzahl des Polypeptidantigens an der Oberfläche der proliferierenden Krebszelle um zumindest 60 % höher ist als an der Oberfläche aller proliferierenden Nicht-Krebs-Zelltypen.

14. Verfahren nach Anspruch 13, worin die Kopienanzahl des Polypeptidantigens an der Oberfläche der proliferierenden Krebszelle um zumindest 80 % höher ist als an der Oberfläche aller proliferierenden Nicht-Krebs-Zelltypen.

15. Verfahren nach Anspruch 14, worin die Kopienanzahl des Polypeptidantigens an der Oberfläche der proliferierenden Krebszelle um zumindest 100 % höher ist als an der Oberfläche aller proliferierenden Nicht-Krebs-Zelltypen.

16. Verfahren nach einem der vorangegangenen Ansprüche, worin die Kopienanzahl des Polypeptidantigens an der Oberfläche einer nicht proliferierenden oder langsam proliferierenden Nicht-Krebs-Zelle um zumindest 10 % höher ist als an der Oberfläche einer proliferierenden Nicht-Krebs-Zelle.

17. Verfahren nach Anspruch 16, worin die Kopienanzahl des Polypeptidantigens an der Oberfläche einer nicht proliferierenden oder langsam proliferierenden Nicht-Krebs-Zelle um zumindest 30 % höher ist als an der Oberfläche einer proliferierenden Nicht-Krebs-Zelle.

18. Verfahren nach Anspruch 17, worin die Kopienanzahl des Polypeptidantigens an der Oberfläche einer nicht proliferierenden oder langsam proliferierenden Nicht-Krebs-Zelle um zumindest 60 % höher ist als an der Oberfläche einer proliferierenden Nicht-Krebs-Zelle.

19. Verfahren nach Anspruch 18, worin die Kopienanzahl des Polypeptidantigens an der Oberfläche einer nicht proliferierenden oder langsam proliferierenden Nicht-Krebs-Zelle um zumindest 80 % höher ist als an der Oberfläche einer proliferierenden Nicht-Krebs-Zelle.

20. Verfahren nach Anspruch 19, worin die Kopienanzahl des Polypeptidantigens an der Oberfläche einer nicht proliferierenden oder langsam proliferierenden Nicht-Krebs-Zelle um zumindest 100 % höher ist als an der Oberfläche einer proliferierenden Nicht-Krebs-Zelle.

21. Verfahren nach einem der vorangegangenen Ansprüche, worin der Schritt des Identifizierens eines Polypeptidantigens den Einsatz von Mikroarrayanalyse umfasst.

## Revendications

1. Méthode pour identifier un antigène polypeptidique sur la surface d'une cellule comme cible pour une thérapie contre le cancer au moyen d'un composé anti-mitotique, la méthode comprenant l'identification d'un antigène polypeptidique en tant qu'antigène ayant un nombre de copies supérieur d'au moins 10 % sur la surface d'une cellule cancéreuse proliférante à celui sur la surface d'une cellule non cancéreuse proliférante du même type cellulaire ;
dans laquelle il existe une différence de moins de 10 % du nombre de copies de l'antigène polypeptidique sur la surface d'une cellule non cancéreuse non proliférante ou à prolifération lente du même type cellulaire, comparativement à la cellule cancéreuse proliférante,
ou dans laquelle le nombre de copies de l'antigène polypeptidique sur la surface d'une cellule non cancéreuse non proliférante ou à prolifération lente du même type cellulaire est supérieur d'au moins 10 % à celui sur la surface de la cellule cancéreuse proliférante ;
dans laquelle la cellule non proliférante est une cellule dans laquelle la phase M du cycle reproducteur de la cellule apparaît dans un délai de moins de 72 heures, et dans laquelle la cellule à prolifération lente est une cellule dans laquelle la phase M du cycle reproducteur de la cellule apparaît dans un délai de moins de 8 heures mais de, ou de plus de, 72 heures ;
ce qui permet d'identifier ledit antigène polypeptidique.

2. Méthode suivant la revendication 1, dans laquelle il existe une différence de moins de 5 % du nombre de copies de l'antigène polypeptidique sur la surface de la cellule non cancéreuse non proliférante ou à prolifération lente, comparativement à la cellule cancéreuse proliférante.

3. Méthode suivant la revendication 1, dans laquelle le nombre de copies de l'antigène polypeptidique sur la surface d'une cellule non cancéreuse non proliférante ou à prolifération lente est supérieur d'au moins 30 % à celui sur la surface de la cellule cancéreuse proliférante.

4. Méthode suivant la revendication 3, dans laquelle le nombre de copies de l'antigène polypeptidique sur la surface d'une cellule non cancéreuse non proliférante ou à prolifération lente est supérieur d'au moins 60 % à celui sur la surface de la cellule cancéreuse proliférante.

5. Méthode suivant la revendication 4, dans laquelle le nombre de copies de l'antigène polypeptidique sur la surface d'une cellule non cancéreuse non proliférante ou à prolifération lente est supérieur d'au moins 80 % à celui sur la surface de la cellule cancéreuse proliférante.

6. Méthode suivant la revendication 5, dans laquelle le nombre de copies de l'antigène polypeptidique sur la surface d'une cellule non cancéreuse non proliférante ou à prolifération lente est supérieur d'au moins 100 % à celui sur la surface de la cellule cancéreuse proliférante.

7. Méthode suivant l'une quelconque des revendications précédentes, dans laquelle l'antigène polypeptidique a un nombre de copies supérieur d'au moins 30 % sur la surface de la cellule cancéreuse proliférante à celui sur la surface de la cellule non cancéreuse proliférante.

8. Méthode suivant la revendication 7, dans laquelle l'antigène polypeptidique a un nombre de copies supérieur d'au moins 60 % sur la surface de la cellule cancéreuse proliférante à celui sur la surface de la cellule non cancéreuse proliférante.

9. Méthode suivant la revendication 8, dans laquelle l'antigène polypeptidique a un nombre de copies supérieur d'au moins 80 % sur la surface de la cellule cancéreuse proliférante à celui sur la surface de la cellule non cancéreuse proliférante.

10. Méthode suivant la revendication 9, dans laquelle l'antigène polypeptidique a un nombre de copies supérieur d'au moins 100 % sur la surface de la cellule cancéreuse proliférante à celui sur la surface de la cellule non cancéreuse proliférante.

11. Méthode suivant l'une quelconque des revendications 1 à 6, dans laquelle ledit antigène polypeptidique a un nombre de copies supérieur d'au moins 10 % sur la surface de ladite cellule cancéreuse proliférante à celui sur la surface de tous les types de cellules non cancéreuses proliférantes.

12. Méthode suivant la revendication 11, dans laquelle ledit antigène polypeptidique a un nombre de copies supérieur d'au moins 30 % sur la surface de ladite cellule cancéreuse proliférante à celui sur la surface de tous les types de cellules non cancéreuses proliférantes.

13. Méthode suivant la revendication 12, dans laquelle ledit antigène polypeptidique a un nombre de copies supérieur d'au moins 60 % sur la surface de ladite cellule cancéreuse proliférante à celui sur la surface de tous les types de cellules non cancéreuses proliférantes.

14. Méthode suivant la revendication 13, dans laquelle ledit antigène polypeptidique a un nombre de copies supérieur d'au moins 80 % sur la surface de ladite cellule cancéreuse proliférante à celui sur la surface de tous les types de cellules non cancéreuses proliférantes.

15. Méthode suivant la revendication 14, dans laquelle ledit antigène polypeptidique a un nombre de copies supérieur d'au moins 100 % sur la surface de ladite cellule cancéreuse proliférante à celui sur la surface de tous les types de cellules non cancéreuses proliférantes.

16. Méthode suivant l'une quelconque des revendications précédentes, dans laquelle ledit antigène polypeptidique a un nombre de copies supérieur d'au moins 10 % sur la surface d'une cellule non cancéreuse non proliférante ou à prolifération lente à celui sur la surface d'une cellule non cancéreuse proliférante.

17. Méthode suivant la revendication 16, dans laquelle ledit antigène polypeptidique a un nombre de copies supérieur d'au moins 30 % sur la surface d'une cellule non cancéreuse non proliférante ou à prolifération lente à celui sur la surface d'une cellule non cancéreuse proliférante.

18. Méthode suivant la revendication 17, dans laquelle ledit antigène polypeptidique a un nombre de copies supérieur d'au moins 60 % sur la surface d'une cellule non cancéreuse non proliférante ou à prolifération lente à celui sur la surface d'une cellule non cancéreuse proliférante.

19. Méthode suivant la revendication 18, dans laquelle ledit antigène polypeptidique a un nombre de copies supérieur d'au moins 80 % sur la surface d'une cellule non cancéreuse non proliférante ou à prolifération lente à celui sur la surface d'une cellule non cancéreuse proliférante.

20. Méthode suivant la revendication 19, dans laquelle ledit antigène polypeptidique a un nombre de copies supérieur d'au moins 100 % sur la surface d'une cellule non cancéreuse non proliférante ou à prolifération lente à celui sur la surface d'une cellule non cancéreuse proliférante.

21. Méthode suivant l'une quelconque des revendications précédentes, dans laquelle ladite étape d'identification d'un antigène polypeptidique comprend l'utilisation de l'analyse en microréseau.
